# EUROPEAN PATENT SPECIFICATION

(11) **EP 1 253 951 B1**
(45) Date of publication and mention of the grant of the patent: **13.10.2004**
(21) Application number: 01907077.0
(22) Date of filing: 07.02.2001
(51) Int. Cl.: A61L 2/18, A61L 2/24, A61B 1/12

(54) **LIQUID CLEANING AND STERILIZATION SYSTEM AND METHOD**
VERFAHREN UND VORRICHTUNG ZUM FLÜSSIG-REINIGEN UND -STERILISIEREN
SYSTEME ET PROCEDE DE NETTOYAGE ET DE STERILISATION PAR DES LIQUIDES

(30) Priority: 07.02.2000 US 499561; 07.02.2000 US 498811; 07.02.2000 US 498869; 31.03.2000 US 193521 P
(43) Date of publication of application: 06.11.2002
(73) Proprietor: STERIS INC., Temecula, California 92590 (US)
(72) Inventor: SANFORD, Bill, R., Willoughby, OH 44094 (US); KRAL, Jude, A., Twinsburg, OH 44087 (US); TVERGYAK, Joseph, Chardon, OH 44024 (US); MOSS, Bernard, J., Willowick, OH 44094 (US); PRIEST, Robert M., Eastlake, OH 44095-3013 (US); HLEBOVY, James, C., Chardon, OH 44024 (US); KELSCH, Daniel, N., Fairview Park, OH 44126 (US); GRESZLER, Alan, J., Elyria, OH 44035 (US); MINEROVIC, David, E., Concord, OH 44077 (US); HOUSTON, John, C., Erie, PA 16506 (US); ROBINSON, Nancy, A., Hudson, OH 44236 (US)
(74) Representative: Gasquet, Denis
(86) International application number: PCT/US2001/003937
(87) International publication number: WO 2001/056615

(56) References cited:
- WO-A-00/23118
- DE-A- 19 858 347
- US-A- 4 552 728
- US-A- 5 225 160
- US-A- 5 279 799
- US-A- 5 558 841
- US-A- 5 858 305

## Description

### Background of the Invention

The present invention relates to the decontamination arts. It finds particular application in connection with an automated system for leak testing cleaning, sterilizing, and drying devices for medical, dental, mortuary, and pharmaceutical applications, and the like, and will be described with particular reference thereto. It should be appreciated, however, that the invention is also applicable to the decontamination of other devices in an automated processing system.

Medical devices, such as endoscopes, and other lumened instruments, are subjected to thorough cleaning and antimicrobial decontamination between each use. During medical procedures, the devices become coated with blood and other protein-rich body fluids. If the instruments are sterilized while they are coated with these materials, the high temperatures and/or chemicals used in the sterilization process tend to cause the materials to set as a hardened layer of biological residue that becomes difficult to remove. Not only do such residues present a barrier to sterilant penetration, but even when sterilized, they may later break down to form toxic substances which pose hazards to patients when the devices are reused.

Traditionally, such devices are often rinsed in a cleaning solution, such as an enzymatic cleaner, to remove the bulk of the blood and other body fluids from their surfaces. The rinsing process is generally carried out manually by immersing the devices in a shallow tray of the cleaning solution. However, for devices such as endoscopes, the cleaning fluid may not penetrate the length of the internal lumen, leaving a portion of the endoscope to become coated with dried body fluids. Additionally, the biological materials and strong cleaners may pose hazards to personnel coming into contact with them.

High temperature sterilization processes, such as steam sterilization in an autoclave, are generally unsuited to the sterilization of endoscopes because of the delicate components and materials from which they are manufactured. The high temperature and pressure tend to curtail the useful life of endoscopes, rubber and plastic devices, lenses, and portions of devices made of polymeric materials and the like. High temperature sterilization alone does not clean. Any body fluids that are not removed prior to thermal sterilization are typically baked on to the instrumentation.

Instruments which cannot withstand the pressure or temperature of the oven autoclave are often microbially decontaminated with gas, such as ethylene oxide gas or hydrogen peroxide vapor. Like steam, gases do not clean, requiring a separate cleaning operation. The ethylene oxide sterilization technique also has several drawbacks. First, the ethylene oxide sterilization cycle tends to be longer than the steam autoclave cycle. Second, some medical equipment can not be sterilized with ethylene oxide gas. Third, ethylene oxide is highly toxic and can present health risks to workers if not handled properly.

Liquid microbial decontamination systems are now utilized for equipment which can not withstand the high temperatures of steam sterilization. Peroxyacetic acid, or peracetic acid, is a useful sterilant and/or disinfectant for a variety of applications, including disinfection of waste and sterilization or disinfection of medical equipment, packaging containers, food processing equipment, and the like. It has a broad spectrum of activity against microorganisms, and is effective even at low temperatures. It poses few disposal problems because it decomposes to compounds which are readily degraded in sewage treatment plants.

In some situations, a technician mixes a disinfectant or sterilant composition with water and then manually immerses the items to be microbially decontaminated in the liquid composition. The high degree of manual labor introduces numerous uncontrolled and unreported variables into the process. There are quality assurance problems with technician errors in the mixing of sterilants, control of immersion times, rinsing of residue, exposure to the ambient atmosphere after the rinsing step, and the like. For sterilizing large, instruments, such as endoscopes with narrow lumens, however, a large receiving tray and a considerable quantity of decontaminant solution are used to accommodate and fully immerse the instruments.

Integrated decontamination systems, such as peracetic acid decontamination systems, have now been developed which provide a premeasured dose of a decontaminant in solution. Items to be sterilized are loaded into a receiving tray of a sterilization system and a cartridge of concentrated decontaminant inserted into a well. As water flows through the system, the decontaminant, which may be accompanied by surfactants and corrosion inhibitors, is diluted and carried to the receiving tray.

The items to be decontaminated are typically loaded into a treatment chamber through an opening closed by a door. It is desirable to maintain a seal between the door and the chamber, to prevent leakage of potentially hazardous sterilization chemicals from the chamber, and also to prevent ingress of potentially contaminated outside air into the chamber once the items are sterile.

Accidental opening of the door during a sterilization cycle poses hazards to operators because of the strong chemicals generally used. Typically, the door includes hinges along one side and a latch mechanism on the opposing side which holds the door securely against the chamber. With large doors, a single latch is often insufficient to maintain a seal along the length of the door. Having multiple latches increases the time required for opening and closing the chamber.

Spraying the exterior of the instruments, while flowing decontaminant solution through the lumens, would have advantages over full immersion of the devices in reducing the quantity of decontaminant solution used. However, because of the complex shape of endoscopes, the spray jets may not reach all of the surfaces of the device. Additionally, interior surfaces of the lumened devices are not reached by the spray. U.S. Patent No. 5,858,305 discloses a decontamination apparatus for medical devices. The device is positioned in a basin and sprayed with a liquid cleaning solution and an antimicrobial liquid from a spray nozzle assembly above the basin. The device is then rinsed with a decontaminated rinse liquid. U.S. Patent No. 5,225,160 to Sanford, et al. discloses a decontamination chamber in which an instrument to be sterilized is supported on a hanger. Spray nozzles spray a sterilant and rinse liquid over the instrument; electroacoustic transducers vary the size of the droplets.

The present invention provides for a new and improved automated system and method for reprocessing endoscopes and the like which overcomes the above-referenced problems and others.

### Summary of the Invention

In accordance with one aspect of the present invention, an automated system for cleaning and microbially decontaminating a device is provided. The automated system includes a cabinet which defines an interior chamber, for receiving the device. Spray nozzles, disposed within the chamber, spray a washing fluid and a microbial decontaminant fluid over an external surface of the device. The system further includes sources of rinse water, the washing fluid, and the microbial decontaminant fluid. A fluid distribution system fluidly connects the sources of rinse water, washing fluid, and microbial decontaminant fluid with the nozzles. A pump is connected with the fluid distribution system for pumping the washing fluid, microbial decontaminant fluid, and rinse water to the nozzles. The pump pumps sprayed solutions to the nozzles. A control system controls the delivery of the washing fluid, microbial decontaminant fluid, and rinse water to the nozzles such that the device is sequentially washed with the washing fluid, microbially decontaminated with the microbial decontaminant fluid, and rinsed with the rinse water. The control system sequentially causes a first set of the spray nozzles to spray the decontaminant solution for a first period of time and then causes a second set of the spray nozzles to spray the decontaminant solution for a second period of time.

In accordance with another aspect of the present invention, a method of cleaning and microbially decontaminating a device is provided. The method includes the sequential steps of positioning the device within a chamber, spraying a washing solution over the device from nozzles within the chamber to remove soil from exterior surfaces of the device; and spraying a microbial decontaminant solution over the device from nozzles within the chamber to microbially decontaminate the exterior surfaces of the device. A rinse fluid is sprayed over the device from nozzles within the chamber to rinse the exterior surfaces of the device. The step of spraying a microbial decontamination solution includes alternately spraying with different groups of nozzles, a first set of the spray nozzles spraying the decontaminant solution for a first period of time and then a second set of the spray nozzles spraying the decontaminant solution for a second period of time.

One advantage of one embodiment of the present invention is that an endoscope or other lumened device is cleaned and microbially decontaminated in a single automated process.

Another advantage of one embodiment of the present invention is that hazards posed to personnel by handling contaminated devices are minimized.

Yet another advantage of one embodiment of the present invention is that a leak resistant closure is created with a single latching mechanism.

A further advantage of one embodiment of the present invention is that the door remains locked during a sterilization cycle.

A yet further advantage of one embodiment of the present invention is that a decontaminant delivery system ensures decontamination of all exterior and interior surfaces of the device being decontaminated.

Another advantage of one embodiment of the present invention is that spraying, rather than fully immersing large items, such as endoscopes, reduçes the quantities of water and decontaminant, pretreatment agents, and cleaning agents used.

Still further advantages of the present invention will become apparent to those of ordinary skill in the art upon reading and understanding the following detailed description of the preferred embodiments.

### Brief Description of the Drawings

The invention may take form in various components and arrangements of components, and in various steps and arrangements of steps. The drawings are only for purposes of illustrating a preferred embodiment and are not to be construed as limiting the invention.
FIGURE 1 is a perspective and diagrammatic view of a cleaning and antimicrobial decontamination processor according to the present invention;
FIGURE 2 is a perspective view of the chamber of FIGURE 1 with the door open;
FIGURE 3 is a plumbing diagram of the system of FIGURE 1;
FIGURE 4 is a front view of the chamber of FIGURE 2;
FIGURE 5 is a sectional view of a section of an endoscope showing spray jets impinging on its outer surface;
FIGURE 6 is a perspective view of the endoscope rack of FIGURES 2 and 4 with an endoscope shown in phantom;
FIGURE 7 is an enlarged perspective view in partial section of a rack peg of FIGURE 6;
FIGURE 8 is a perspective view of one embodiment of an endoscope clip;
FIGURE 9 is a perspective view of the endoscope clip of FIGURE 8 showing the fingers in partial section;
FIGURE 10 is a perspective view of another embodiment of an endoscope clip;
FIGURE 11 is an enlarged side sectional view of the valve reprocessor of FIGURE 3;
FIGURE 12 is an enlarged top view of the door latching and locking mechanism of FIGURE 1 with the door partially open;
FIGURE 13 is a sectional view of the door latching and locking mechanism of FIGURE 12 with the door closed;
FIGURE 14 is a top view of the door latching and locking mechanism of FIGURE 12 with the door closed and the latching mechanism engaged;
FIGURE 15 is a side view of the cabinet of FIGURE 1;
FIGURE 16 is an enlarged perspective view of the cylinder and piston of the moveable rack activation system of FIGURE 1;
FIGURE 17 is a side view showing the positions of the rack of FIGURE 16 before and after (phantom) actuation of the piston;
FIGURE 18 is an enlarged side view showing the activation system prior to activation and/or after deactivation;
FIGURE 19 is an enlarged side view showing the activation system after activation;
FIGURE 20 is a plot showing endoscope pressure, fluid temperature, and peracetic acid concentration with time for a washing and microbial decontamination cycle in the processor of FIGURE 1; and
FIGURE 21 is a perspective view of a twin cabinet embodiment of a processor in accordance with the present invention.

### Detailed Description of the Preferred Embodiments

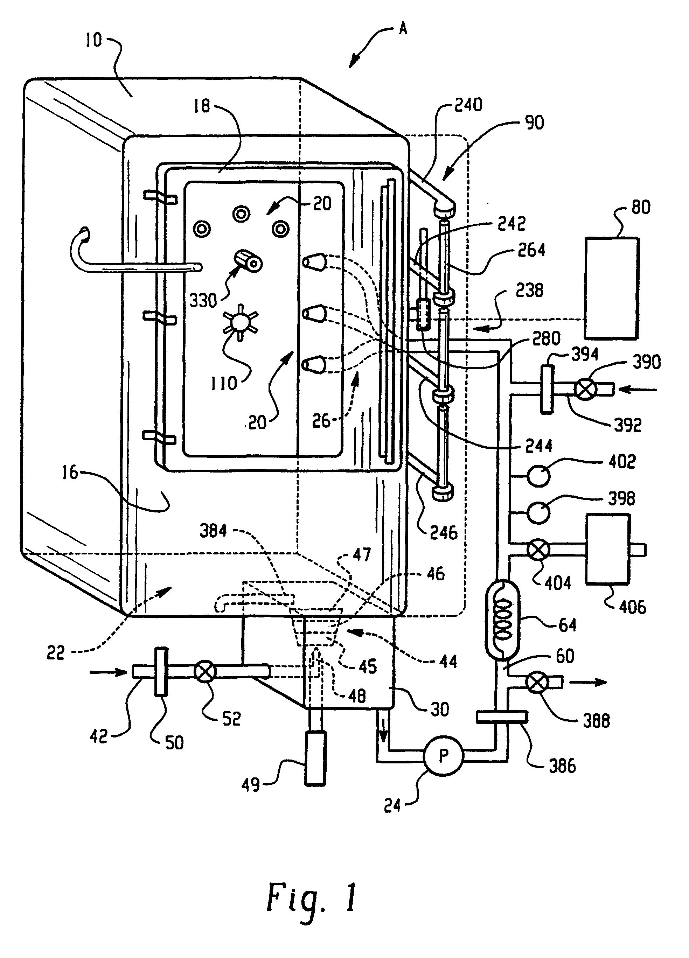

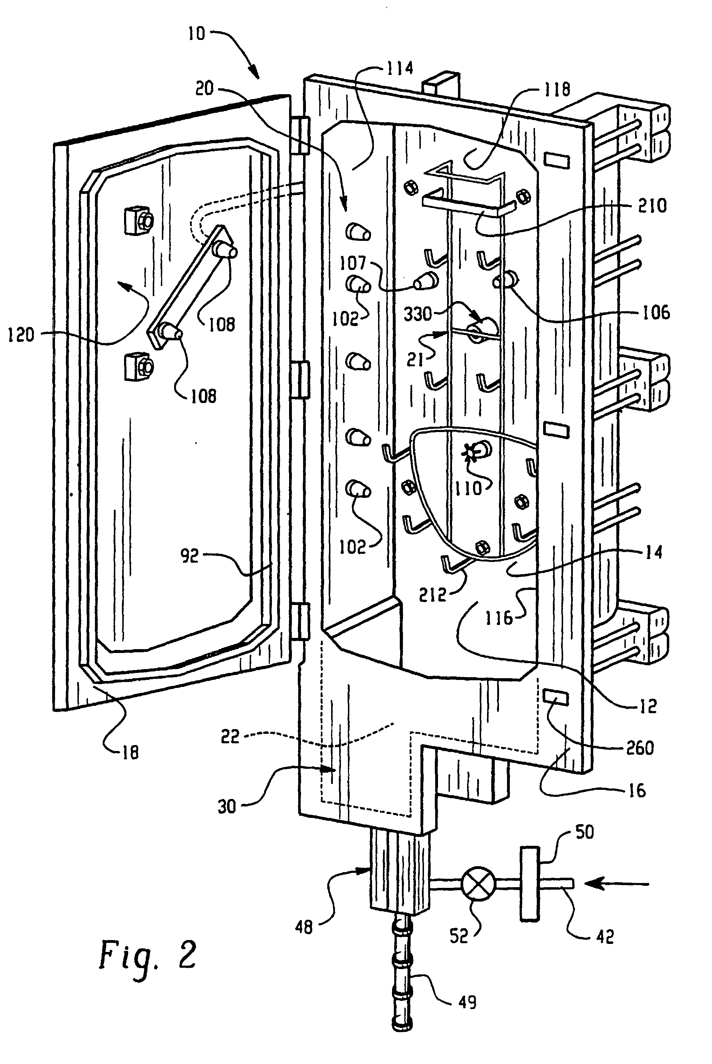

With reference to FIGURES 1 and 2, an automated liquid cleaning and antimicrobial decontamination processor or system A sequentially leak tests and washes then sterilizes or disinfects items, such as medical, dental, and pharmaceutical devices, and the like. While particular reference is made to the cleaning and microbial decontamination of lumened instruments, such as endoscopes, it is to be appreciated that the processor A has application in the cleaning and/or decontamination of a variety of different devices. The processor A is particularly suited to the cleaning and microbial decontamination of instruments which are heat labile, i.e., those which, because of their components or materials, may be damaged by temperatures over about 60°C.

The term "endoscope," as used herein, should be understood to include a wide variety of lumened instruments, including angioscopes, artheroscopes, laparoscopes, bronchoscopes, duodenoscopes, catheters, and the like.

The term "microbial decontamination" and other terms relating to decontaminating will be used herein to describe sterilization, disinfection, and other antimicrobial treatments which are designed to destroy microorganisms contaminating the items. The term "washing" will be used herein to describe the physical removal of soil from the items, without necessarily destroying the microorganisms contaminating the items.

The processor **A** includes at least one combined washing and microbial decontamination cabinet 10 which defines an interior washing and microbial decontamination chamber **12**.

Items to be washed and microbially decontaminated are loaded into the chamber **12** through an opening **14** in a vertical front wall **16** of the cabinet, closed by a door **18.** Within the chamber, a fluid delivery system **20,** comprising spray jets and connection nozzles, sprays a washing/decontaminant solution over exterior surfaces of the items and directs the solution through internal passages of endoscopes and other objects with lumens. A rack **21** supports one or more endoscopes in a suitable position for optimal effective washing and decontamination by the spray system **20.** The endoscope may be loaded on to the rack prior to loading into the chamber, or the rack may be positioned in the chamber prior to attachment of the endoscope.

A collection tank or sump **22** forms the base of the cabinet **10** and receives the sprayed washing/decontaminant solution as it drips off the items. A high pressure pump **24** delivers the washing/decontaminant solution under pressure to the spray system **20** through a fluid distribution system or manifold **26**.

A well or mixing chamber **30** sequentially receives doses of a cleaner concentrate and a concentrated decontaminant. The cleaner concentrate mixes with water to form a washing solution for cleaning the items prior to antimicrobial decontamination. The concentrated decontaminant is preferably an antimicrobial agent or comprises reagents which react to form an antimicrobial agent on mixing with water. The cleaner concentrate may be an enzymatic cleaner, or an acid or alkaline cleaner, and may include detergents, surfactants, and the like. A preferred cleaner concentrate is a pH neutral, low foaming composition, which is not harmful to the components of the device. The cleaner concentrate and concentrated decontaminant may be in solid or in liquid form. As shown in FIGURES 1 and 2, the well **30** is integral with the collection tank **22** of the chamber, although a separate well is also contemplated.

A preferred antimicrobial agent is peracetic acid, either in concentrated liquid form, or as a reaction product of powdered reagents, such as acetyl salicylic acid and sodium perborate. other peracids, or mixtures of peracids are also useful antimicrobial agents. A water inlet **42** supplies water, typically from a municipal water system, to the well **30.** The water mixes with detergents, surfactants, corrosion inhibitors, pH buffers, the concentrated decontaminant, and other selected components in the well to form wash, decontaminant, or other solutions.

Preferably, the concentrated decontaminant, cleaner concentrate, and the corrosion inhibitors, buffers, and other components are supplied in a disposable package or cup **44** which is positioned in the well **30** prior to a decontamination cycle. The cup **44** separately holds the measured doses of the cleaner concentrate, a pretreatment mixture of buffers, surfactants, corrosion inhibitors, and other pretreatment chemicals, and the concentrated decontaminant in separate compartments **45, 46,** and **47,** respectively, for separate release into the system. In this way, the items are first washed and then microbially decontaminated. A cup cutter **48**, or other suitable opening member, driven by an drive system, such as an air cylinder **49** is positioned at the base of the well **30** for opening selected compartments of the cup.

The quantity of water entering the system is regulated to provide a washing/decontaminant solution of a desired concentration in the decontamination chamber **12**. The water is preferably passed through a microporous filter **50** in the water inlet line **42,** which filters out particulates. Optionally, a 5 centimeter filter may be provided to remove microbes. A valve **52** in the water inlet **42** closes when the selected quantity of water has been admitted.

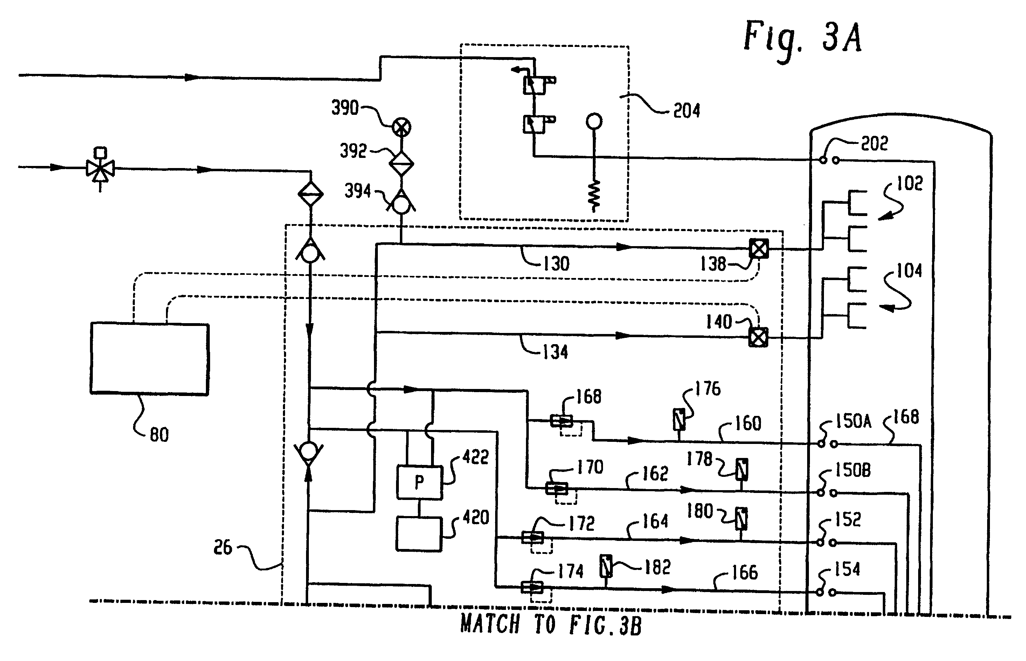

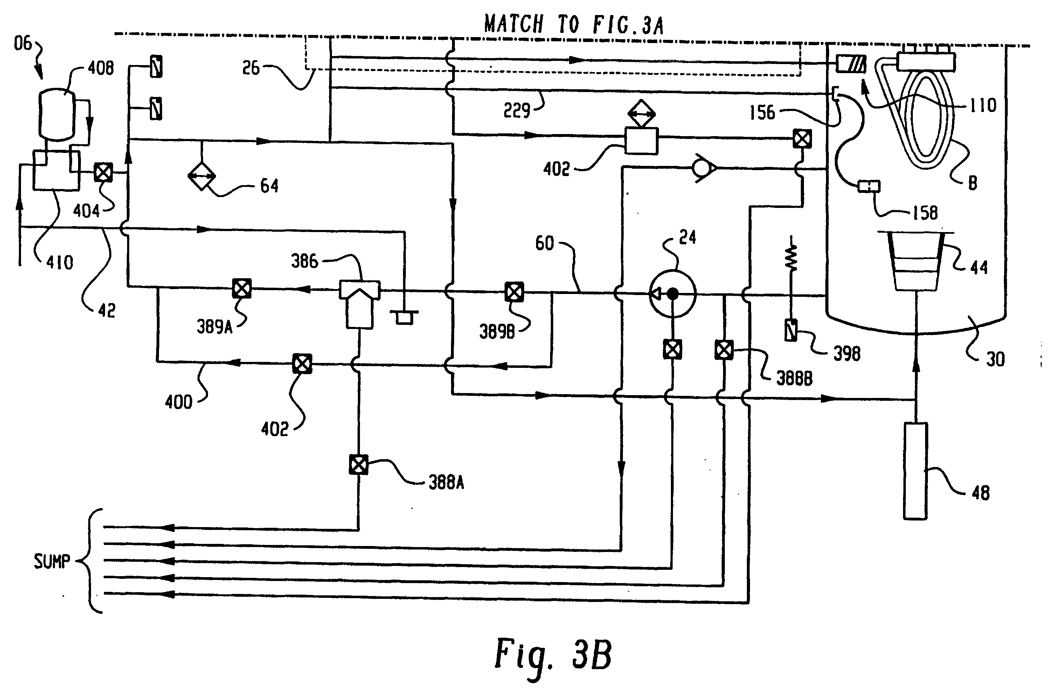

With reference also to FIGURE 3, a fluid supply pathway **60** connects the well **30,** the pump **24,** and the fluid distribution system **26.** Thus, a fluid circulation loop is provided which circulates the washing and decontaminant solutions through the well **30,** pathway **60,** fluid distribution system **26,** and spray system **20.** Sprayed solutions collect in the well and are pumped by the pump **22** through the pathway, fluid distribution system, and back to the spray system **20.** A heater **64,** situated in the fluid supply pathway **60,** heats the decontaminant solution and optionally the washing solution and a rinse liquid to a preferred temperature(s) for effective cleaning, decontamination, and rinsing.

A computer control system **80** controls the operation of the processor **A,** including the pump **24,** the heater **64,** the valves **52,** locking of the door **18,** and the like. The control system **80** may control one or more additional systems **A**, if desired.

A door latching and locking mechanism **90** holds the door in the closed position against the front face of the cabinet and prevents the opening of the door during a washing and decontamination cycle. A seal member **92**, such as a gasket, is positioned between the door and the front face **16** of the cabinet to provide a fluid tight seal at the pressures used in the cabinet.

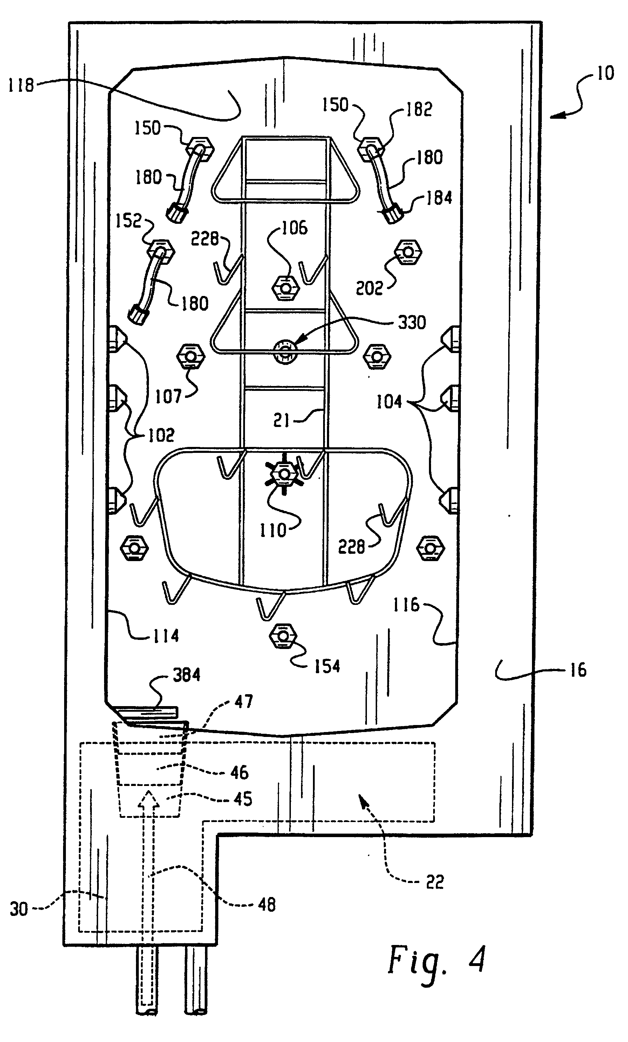

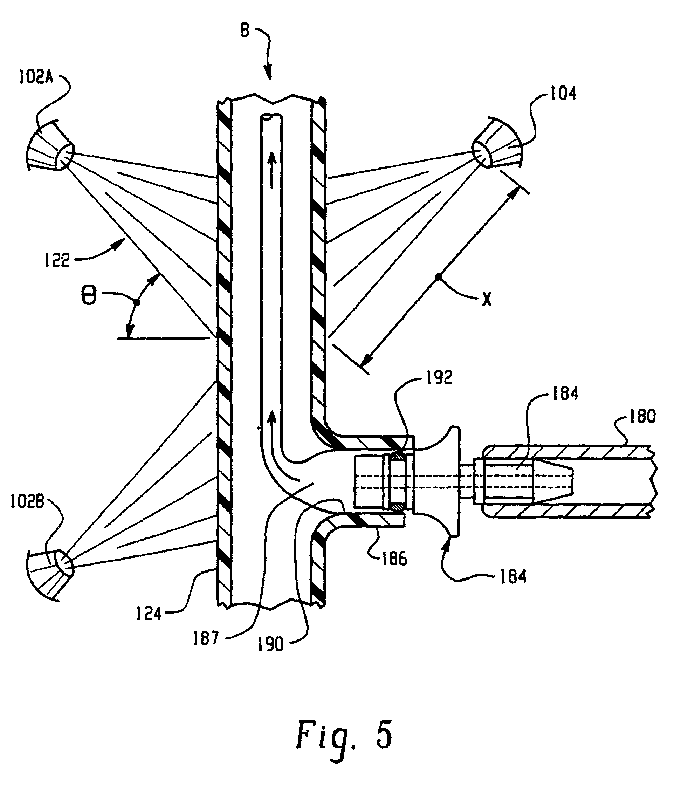

With reference to FIGURES 2 and 3, and also to FIGURE 4, the spray system 20 includes several types of spray nozzles **102, 104, 106, 107, 108,** and **110,** which direct the cleaning/decontaminant solutions over an endoscope **B** and other items within the chamber **12** for complete coverage. The pump supplies the nozzles with the washing/decontaminant fluid at a pressure of about 60-80 psi (4.2-5.6 Kg/sq.cm). The spray nozzles **102** and **104** are located on left and right side walls **114, 116** of the chamber **12,** respectively. These have a spray angle of preferably about 90°, for impacting the surfaces of the endoscope at high pressure. The spray nozzles **106** are located on a rear wall **118** of the chamber. These nozzles spray over a wider angle, preferably about 120 degrees, for wider coverage, although with lesser impact than the nozzles **102, 104.** The spray nozzle **107** extends forward from the rear wall. It has a narrow spray angle of 45 degrees and is aimed to directly impact a contact point on the device. The spray nozzles **108** are attached to an inner surface **120** of the chamber door **18.**

The spray nozzle **110** extends forwardly from the rear wall **118** of the chamber and directs cleaning fluid radially in multiple directions for wide coverage. As shown in FIGURE 4, the nozzle **110** includes multiple spray heads. Six spray heads are shown, angled at 60 degrees apart, for a 360 coverage. Alternatively, spray nozzle **110** is a rotating nozzle, which is rotated through a 360 degree path to deliver solution in many directions.

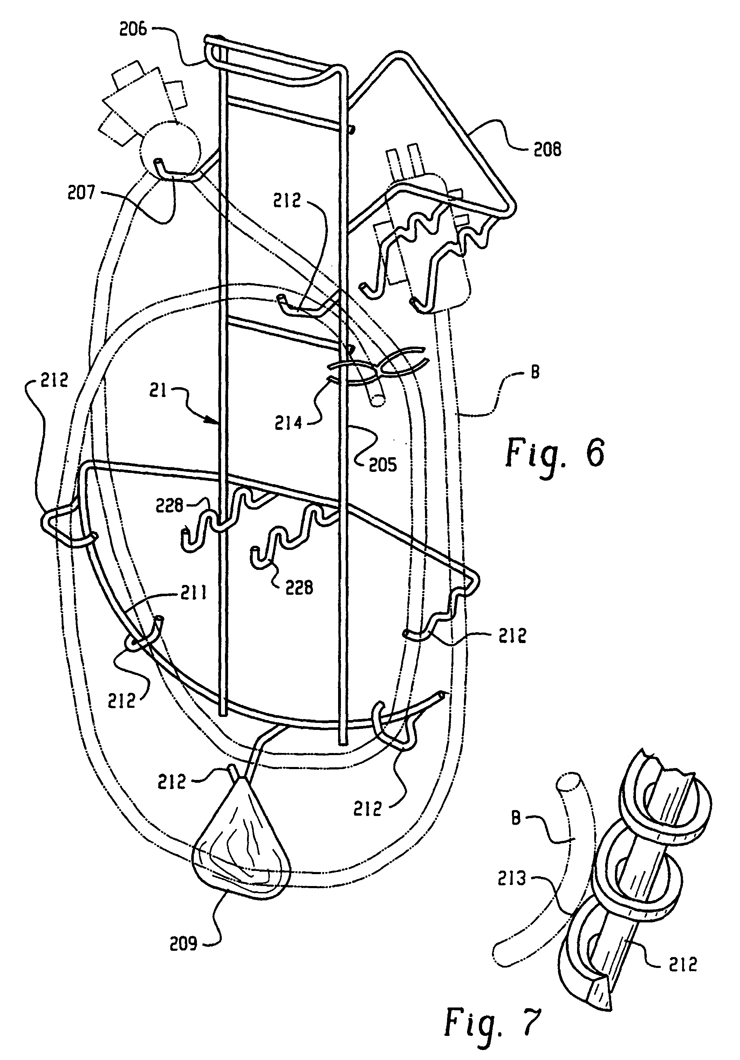

With reference also to FIGURE 5, the spray nozzles **102, 104, 106, 108** are angled such that all surfaces of the endoscope B are contacted by the spray of decontaminant solution emitted from the nozzles. Specifically, each nozzle spray jet **122** strikes the endoscope surface **124** at a shallow angle θ, relative to normal to the endoscope surface. Preferably, the angle θ is less than about 45 degrees, i.e., each surface of the endoscope is struck with at least one spray jet at an angle of no more than about 45 degrees from normal. Thus, the nozzles are angled to deliver the decontaminant/cleaning solutions at different angles. For example, as shown in FIGURE 5, nozzle **102A** is directed downwardly, while nozzle **102B** is directed upwardly.

Additionally, each surface of the endoscope is no more than a maximum distance **x** from the closest spray nozzle, so that the endoscope receives the full force of the spray jet. Preferably, **x** is no more than 20 centimeters, more preferably, **x** is less than about 15 centimeters. Further, each surface of the endoscope is no less than a minimum distance from the closest spray nozzle, so that the endoscope receives the full force of the spray jet. Preferably, the minimum distance is at least 5 centimeters.

With reference once more to FIGURE 3, to obtain these minimum criteria, the nozzles are in many cases positioned so closely that their sprays may interact. The interaction, prior to contacting the instrument, can negate or alter their force, angle of impact and other characteristics. To avoid the spray jets **122** from different directions canceling each other out, the jets are pulsed in sequence. For example, the manifold **26** includes a first fluid line **130** which supplies nozzles **102** and a second fluid line **134** which supplies nozzles **104.** The controller **80** sequentially opens an air diaphragm valve 138 in the first line **130** for a few seconds, allowing the cleaning/decontaminant and rinse solutions to flow to nozzles **102,** then closes valve **138** and opens an air diaphragm valve **140** in the second line **134** for a few seconds, allowing the cleaning/decontaminant solution to flow to nozzles **104.**

With reference now to FIGURES 2 and 3, the spray system **20,** in addition to the nozzles, also includes several connection ports **150, 152,** and **154,** for supplying washing/decontaminant solution to the internal passages of the endoscope **B** and an associated set of biopsy forceps. An additional port **156** may be provided for supplying the solutions to a valve reprocessor **158.** The different internal passages of a typical endoscope and biopsy forceps are rated to withstand different maximum pressures. The connection ports supply washing/ decontaminant solution at an appropriate pressure that is below the maximum pressure rating for the passage to which the connection port supplies solution. For example, as shown in FIGURE 3, the manifold includes fluid lines **160, 162,** which supply fluids to connection ports **150A** and **150B** at a first pressure, preferably of no more than about 1.4 Kg/sq.cm, for washing/decontaminating the lumens, and line **164,** which supplies connection port **152** at a second pressure, preferably of no more than about 210 mmHg (2.8 Kg/sq.cm), for washing/decontaminating elevated guide wire passages. Another fluid line **166** supplies connection port **154** at a third pressure, preferably of no more than about 210 mmHg (2.8 Kg/sq.cm), for cleaning/decontaminating the biopsy forceps. Pressure regulators **168, 170, 172,** and **174** in each of the fluid lines **160, 162, 164,** and **166** are set to ensure that the maximum pressure is not exceeded. Pressure switches **176, 178, 180, 182** detect the presence of a pressure drop in the lines **160, 162, 164,** and **166.**

With reference once more to FIGURES 4 and 5, the connection ports **150, 152,** and **154** are connected with the respective internal passages of the endoscope and biopsy forceps by tubes **180,** each with a quick connect **182** at the connection port end and a suitable connector **184** at the other end for connecting with the inlet port **186** of the respective internal passage, for releasably and quickly connecting the fluid lines with the respective internal passages **187.** To avoid confusion and accidental over-pressurization of the various lumens **187,** the quick connects **182** for the low pressure lines **160, 162, 166** will not connect with the high pressure connection port **152**. In the preferred embodiment, the connectors **182** have different sizing; but, different shapes and the like are also contemplated.

The connectors **184** are preferably leaking connectors, i.e., they allow a controlled portion of the washing/decontaminant solution to flow between the connector and the inlet port to contact all adjacent surfaces **190** of the inlet port **186.** This ensures that all the accessible surfaces of the internal passage **187** are contacted with the washing/decontaminant solution. The relative flow is balanced for optimum cleaning of all points. The majority of the solution travels along the full length of the endoscope internal passage and out of the endoscope into the chamber **12.**

In the embodiment of FIGURE 5, the leaking connector **184** includes a metal C-ring **192.** The C-ring is seated loosely in an annular groove in a portion of the connector which is received past a small lip of the inlet port **186.** The ring spaces the connector from the internal surfaces **190** of the inlet port, allowing a portion of the fluid to flow around it and out of the inlet port **186**. Other configurations of male and female leaking connectors are also contemplated. Analogous plug members with controlled leakage at the interconnection are used to plug selected ports.

With reference to FIGURE 3, a further connection port **202** in the chamber connects a leak detector **204** with the venting connector port of the endoscope for testing the endoscope for leaks. The leak detector supplies air under pressure to the venting connector port and its associated internal passage for detecting leaks from the internal passage. If leaks are found, the leak detector aborts the cycle to prevent fluids from leaking into sensitive regions of the scope.

With reference once more to FIGURE 2 and reference also to FIGURE 6, the rack **21** is preferably removable from the chamber **12.** To accommodate different types of endoscopes, several racks **21** are provided, each one configured for receiving a particular type or family of endoscopes. The appropriate rack is selected according to the endoscope to be reprocessed, and the endoscope fitted to the rack prior to or after hooking or otherwise attaching the rack within the chamber. The shape of the rack is configured for correctly positioning the endoscope in the chamber so that the most difficult parts of the endoscope to be cleaned (e.g., the control head and light guide end) are reproducibly positioned so that they receive cleaning and antimicrobial solutions from the spray jets at optimal angles. This avoids the need to change the angles of the spray jets to achieve optimal cleaning for each different type of endoscope. The spray jets are orientated so the most difficult to clean portions are fully cleaned. If desired, the rack may be configured to support two or more endoscopes.

The rack includes a central rectangular support frame **205** with a carrying and connecting handle **206** attached at an upper end thereof. Mounted on the frame are support members **207, 208,** which are configured for receiving the endoscope operating section and light guide connector sections, respectively. Small, separate components of the endoscope, such as hoods, plugs, and other semi-reusable items, may be hung from the rack in a porous bag **209.** The upper end of the rack is releasably mounted on a suitably receiving member or members **210** within the chamber.

The rack includes an arcuate portion **211** which supports a number of pegs or tabs **212**. The pegs on the arcuate section and the support frame **205** define a circle for support of the flexible tubes (the umbilical cable and the insertion tube) of endoscope **B** such that the tubes curve in a wide loop on the rack **21.** Preferably, the rack and hooks position the endoscope such that it is not bent sharper than its minimum bend radius, typically about 15 centimeters. In the preferred embodiment, the bend radius is at least 18 centimeters, i.e., no portion of the flexible portions of the endoscope tubes are bent into a curve which has a radius of less than about 18 cm. This ensures that as the endoscope is wrapped around the pegs **212** it is correctly positioned for receiving the full force of the spray jets and that there are no inaccessible or potentially damaging tight bends in the endoscope. Depending on the stiffness of the flexible tube, the tube is mounted inside and/or over the pegs. The pegs are positioned at angular intervals such that the end of the tube of every endoscope in the family ends up near, but just beyond, one of the pegs. A single endoscope rack is thus able to accommodate and correctly position the tubular portion of any one of a family of endoscopes having different insertion tube/umbilical cable lengths on the pegs, while correctly positioning the control head and light guide end at the upper end of the rack.

The rack is preferably formed from stainless steel or other materials which are resistant to the decontaminant solution and other chemicals employed in the chamber.

To minimize contact with the endoscope, and improve access of the spray of washing or decontaminant solutions to the contact areas, the support members **207**, **208**, and pegs **212**, preferably make only "point contact" with the endoscope, i.e., the area of contact is as small as is possible, without resulting in damage to the endoscope. In one preferred embodiment, the pegs and support members are formed from a screw-threaded stock, which contacts the endoscope only at tips **213** of the threads, as shown in FIGURE 7. Preferably, the tips of the threads are blunted, such as acme threads or threads with a sinusoidal or other curved cross section, to avoid indentation, scratching, or other damage to the endoscope. A clip **214** clips to the rack and provides a loosely constraint to the endoscope tip.

The rack is preferably formed from stainless steel, or formed from other materials, which are resistant to the decontaminant solution and other chemicals employed in the chamber.

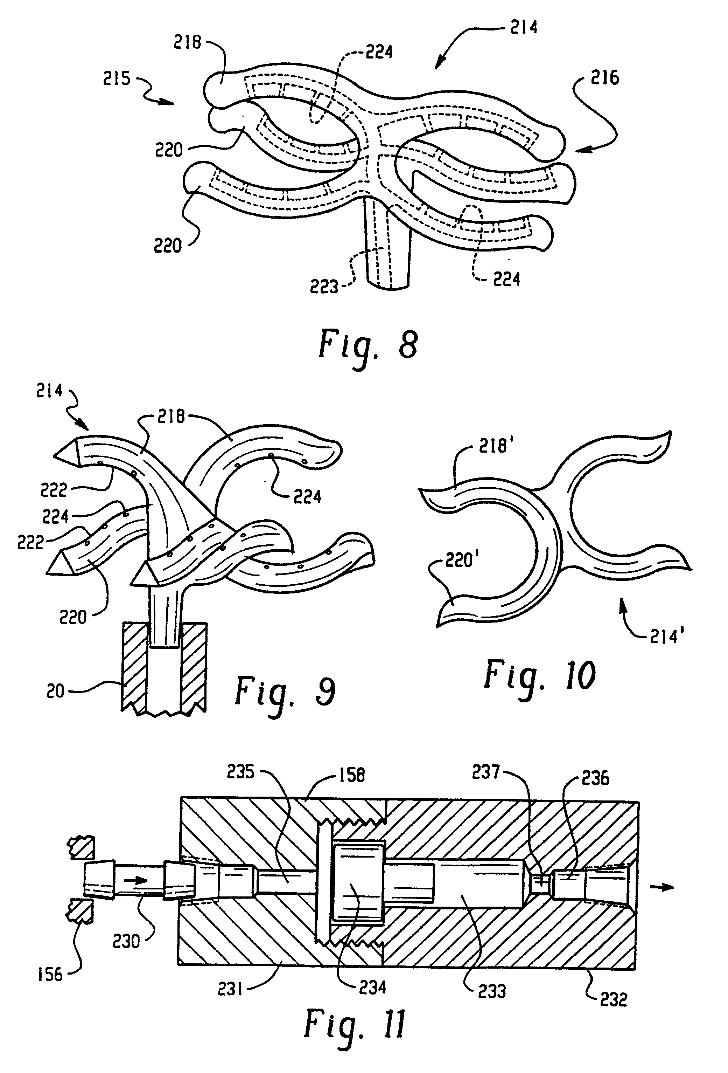

With reference also to FIGURES 8 and 9, one or more clips **214** is attached to the tip of the endoscope insertion tube, or other flexible, tubular portion of the endoscope, to prevent it swaying and breaking during transport or during the cycle. The clip includes a first gripping portion **215**, which releasably grips the tip of a flexible portion of the endoscope **B** and a second gripping portion **216**, which releasably grips another portion of the endoscope or the rack **19**. Each of the gripping portions includes at least one upper finger **218** and at least one lower finger **220.** The clip **214** of FIGURES 8 and 9 includes one upper finger and two, spaced lower fingers. FIGURE 10 shows an alternative embodiment of a clip **214',** where each gripping portion includes one upper finger **218'** and one lower finger **220'.** Other embodiments of the clip are also contemplated. For example, the clip could be permanently attached to the rack **21** and have only a single gripping portion for gripping the endoscope tip.

The clip **214, 214'** is preferably formed from a resiliently flexible material, such as Nylon or Delrin™. Accordingly, when the tubular portion to be gripped (e.g., the endoscope tip or rack) is pressed against the tips of the fingers **218, 220,** the upper and lower fingers are splayed apart, allowing the tubular portion to be inserted therebetween. The fingers **218, 220** then snap back to grip the tubular portion firmly, but not so tightly that access of the washing and decontaminant solutions is prevented. The material selected for forming the clip is also one which is resistant to the chemicals used in the washing and microbial decontamination system.

With particular reference to FIGURE 8, it is important to minimize the contact area between the clip **214, 214'** and the endoscope tip to ensure complete sterilization of the outer surfaces of the endoscope. To achieve this, the fingers **218** and **220** have a triangular cross section with a ridge **222** of the triangle, of very small radius, in contact with the endoscope (essentially point contact). This reduces shadowing, i.e., the interference of the clip with the spray jets. Additionally, providing two, spaced apart lower fingers **220** allows the solutions to contact the endoscope tip between the fingers while maintaining a firm grip on the tip. To avoid damage to the endoscope, the contacting ridge **222** is slightly rounded rather than defining a sharp point. The shape of the ridge is optimized to minimize contact while avoiding damage or indentations in the tip.

Optionally, the clip **214** is fluidly connected with the fluid distribution system **26.** A fluid pathway **223** inside the clip selectively connects the fluid distribution system with apertures **224** defined in the ridges **222**. The washing and decontamination fluids flow out of the apertures **224** and over the surfaces of the endoscope in contact with the clip which otherwise may escape the full force of the spray jets from the nozzles.

Preferably, several interchangeable clips **214** of different dimensions are provided so that an appropriate clip may be selected according to the dimensions of the endoscope/tip.

Optionally, the rack **21** includes support members **228,** for supporting coiled biopsy forceps, which are designed to pass through a channel of the endoscope, or other accessories to be cleaned and decontaminated. To anchor the forceps more securely, they are preferably coiled on a carrier which is supported on pegs **228.**

The rack **21** and clip(s) **214** are designed to hold the endoscope firmly to avoid damage, but yet allow a small amount of movement (i.e., wobbling) of the endoscope during processing, facilitated by the pulsing of the spray jets. This movement allows access of the solutions to those areas of the endoscope making contact with the rack pegs, support members and clips to ensure that the entire exterior surface of the endoscope is thoroughly cleaned and microbially decontaminated.

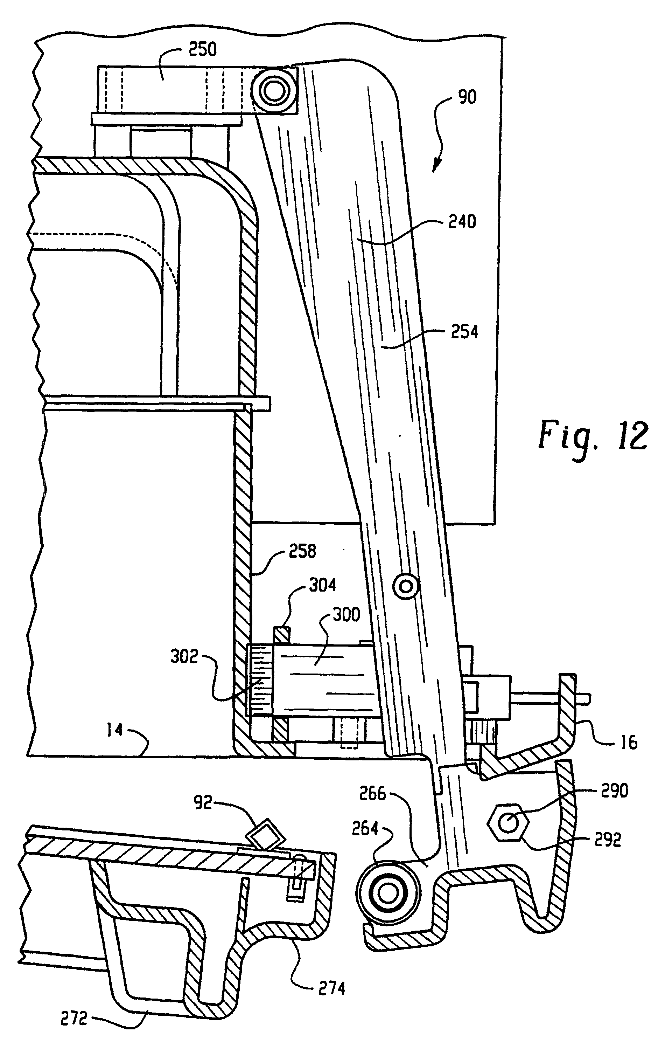

With reference now to FIGURES 3 and 11, a fluid supply line **229** connects the fluid distribution system with the connection port **156** for the valve reprocessor **158.** The valve reprocessor **158** is releasably connected to connection port **156** within the chamber by a quick connect **230**, or other suitable connection member. The valve reprocessor includes cap and base portions **231** and **232,** respectively, which are threadably connected to each other to define an interior chamber **233**, and inlet and outlet passages **235, 236.** The chamber receives an endoscope valve **234** for reprocessing. The cap **231** is sized such that, when the cap is threadably connected to the base **232**, the valve head is compressed to its open position. This allows the washing and decontaminant solutions to flow over and through the valve **234.** A restriction **237** in the outlet passage maintains the solutions under pressure as they pass through the valve.

With continued reference to FIGURE 1 and reference also to FIGURES 12, 13, and 14, the door latching and locking mechanism **90** includes a latching mechanism **238,** which holds the door **18** closed. The latching mechanism **238** includes at least two and preferably four latching arms **240,242,244,246.** Each of the latching arms is pivotally connected to a clevis **250,** which is rigidly mounted to a rear wall **252** of the cabinet. Each of the arms includes a flat plate **254,** which is pivotally connected to the clevis at a pivot point **256.** The plate **254** extends horizontally forwardly from the pivot point **256**, adjacent a side wall **258** of the cabinet. The latching arms **240,242,244,246** extend forwardly of the front face **16** of the cabinet, through suitably positioned slots **260** in the front wall. FIGURE 2 shows the slots, but with the latching arms omitted for clarity. One or more rollers **264** (three are shown in FIGURE 1) is vertically mounted between pairs of forward ends **266** of the latching arms. The rollers **264** rotate about a vertical axis.

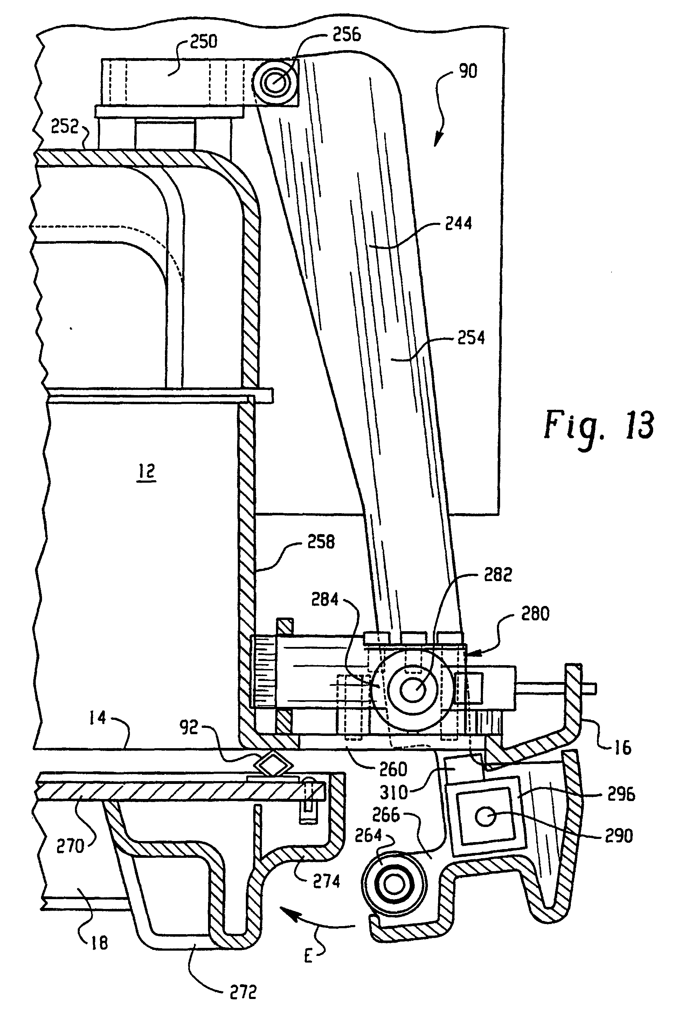

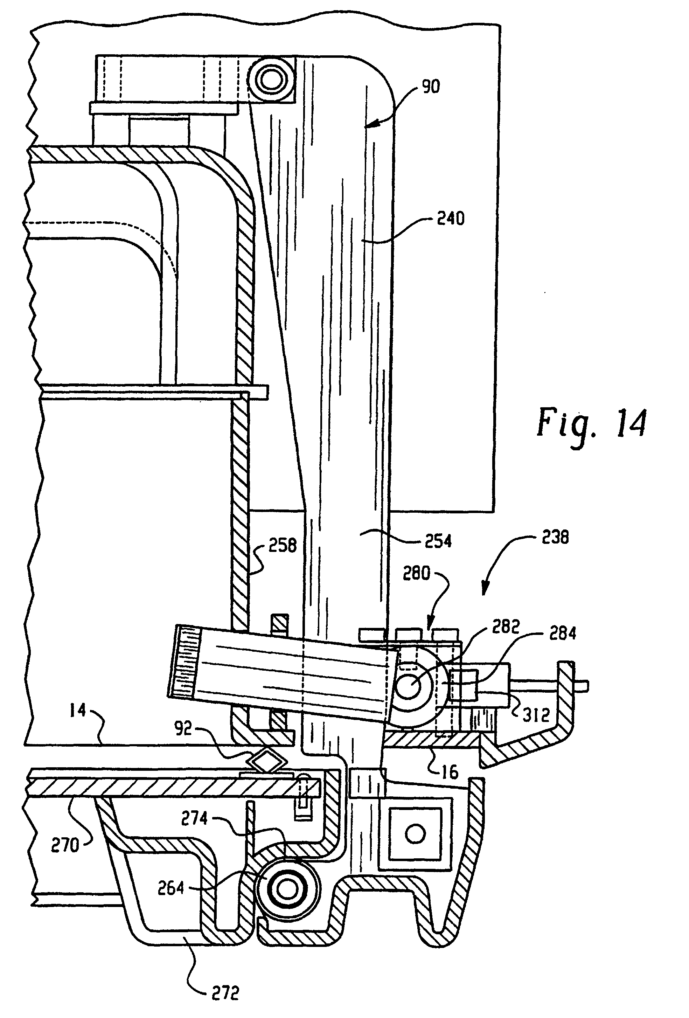

Vertically mounted on a front face **270** of the door, adjacent the door latching mechanism **238,** is an engagement member **272** with a vertically extending camming surface **274** having an L-shaped cross section. When the door **18** is in the closed position, as shown in FIGURE 13, the latching mechanism 238 can be manually, or automatically pivoted about the pivot points **256** in the direction of arrow **E** from the disengaged position until the rollers **264** engage the camming surface **274.** The camming surface is preferably formed from rubber or other suitable rigid material. In the engaged position (FIGURE 14), the rollers **264** hold the door **18** firmly against the front face **16** of the cabinet. In this position, the compression seal **92** is compressed between the door and the cabinet, creating a seal around the opening **14** of the chamber.

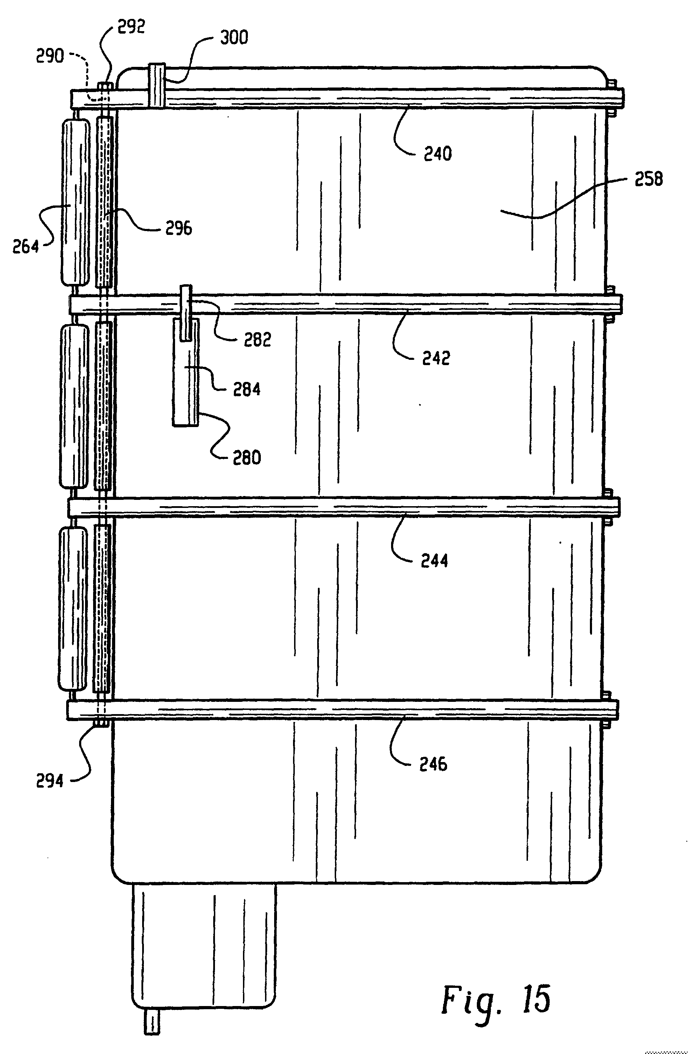

With reference to FIGURES 1, 13, 14, and also to FIGURE 15, the door latching and locking mechanism **90** also includes a locking mechanism **280,** which is actuated by the control system **80** when the latching arms **240,242,244,246** are in the latched position (FIGURE 14). The locking mechanism **280** preferably includes a piston rod **282** actuated by an air cylinder **284.** In the locked position, the rod **282** extends vertically upward from the air cylinder and engages at least one of the latch arms **242** as shown in FIGURE 15. This prevents outward movement of the latch arm and disengagement of the rollers **264** from the engagement member **272**. The rod is retracted to an unlocked position before the latching mechanism **238** can be disengaged and the door **18** opened.

Preferably, the latching mechanism **238** includes a supporting member **290,** such as a vertically extending wire shaft which connects each of the latching arms **240, 242, 244, 246** together as shown in FIGURE 15. The shaft **290** passes through suitably positioned apertures and each of the latching arms in turn and is held in tension by upper and lower nuts **292, 294.** Blocks **296,** each having a central bore, are mounted on the shaft, between pairs of the latching arms, to space the latching arms a suitable distance apart. Thus, the supporting member ensures that each of the latching arms moves generally together, while allowing a limited amount of relative freedom of movement to compensate for minor differences in the width of the door, and the like.

With particular reference to FIGURE 12, which shows the door in the partially open position, a latch arm stop **300** is mounted on at least one of the latching arms **240**. The latch arm stop **300** extends horizontally from adjacent the forward end of the latching arm, towards the side **258** of the cabinet and includes a downwardly protruding stop **302**, formed from rubber or other resilient material. The stop limits the outward movement of the arm by engaging a rearwardly extending flange **304** which is connected to the front wall **16** of the cabinet.

With particular reference to FIGURE 13, a sensor system detects whether the door is properly latched and locked. The sensor system includes a first sensor **310,** mounted on the forward end **266** of one of the latching arms, which senses that the latching arm is properly positioned adjacent the engagement member **272.** The sensor **310** signals the control system **80** when the sensor is closely spaced from the engagement member **272,** as shown in FIGURE 14. A second sensor **312** forms a part of the locking mechanism. The second sensor detects whether the piston rod **282** is extended, and therefore engaging the latch arm plate **254,** and signals the control system.

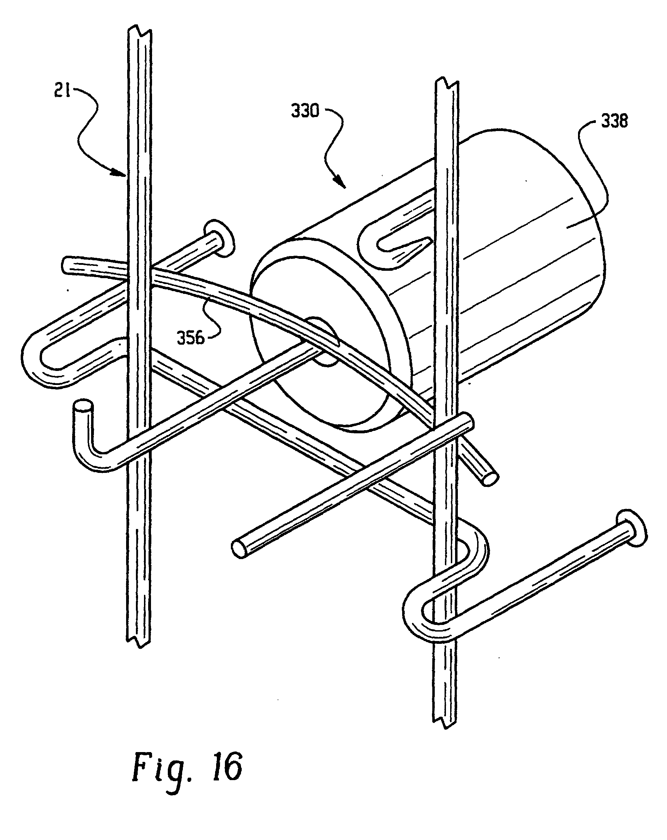

With reference now to FIGURES 1 and 16-19, the endoscope rack is agitated, throughout at least the sterilization portion of the cycle, by a support activation system **330.** The activation system is mounted such that it contacts the rack and provides a pulsing movement which causes the rack to vibrate or move, shaking the endoscope slightly in the process. In this way, the position of the endoscope changes frequently and the positions of the contact points also change.

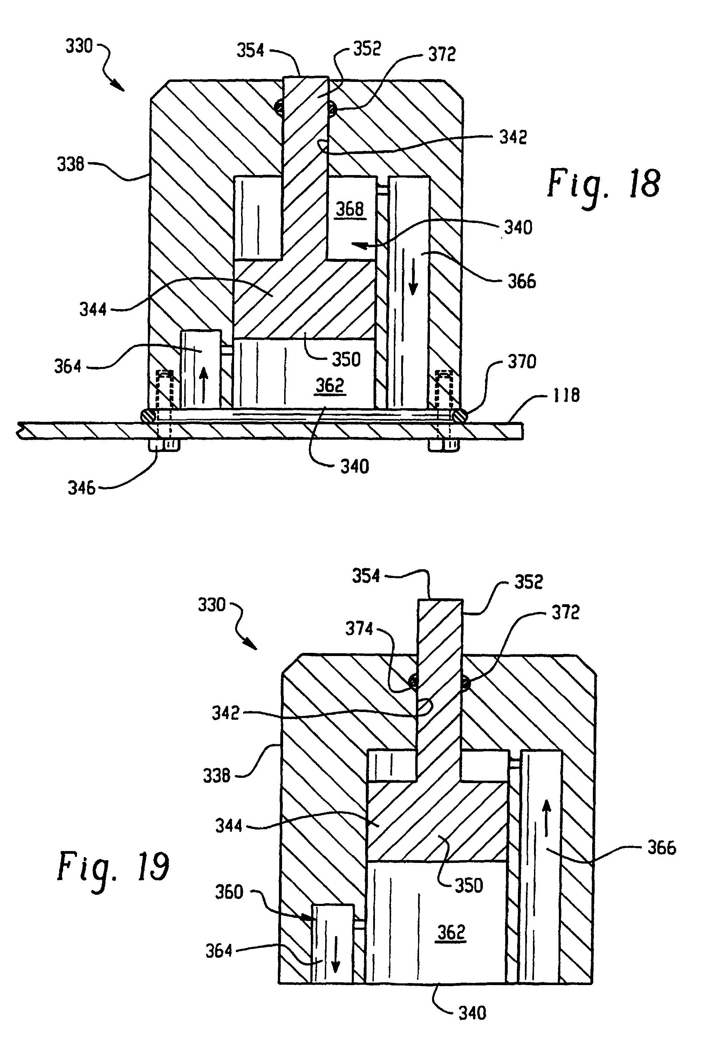

The system **330** includes a housing or cylinder **338,** which defines an internal cavity **340** having an opening or bore **342** in a forward end thereof. A piston **344** is received by the housing. The housing is affixed adjacent a rearward, open end **344** to the rear wall of the chamber **12** by screws **346** or other suitable fixing members. The piston **344** includes a cylindrical portion **350,** which reciprocates within the cavity, and is shaped for sliding engagement with the walls of the cavity. A shaft **352** extends forwardly of the cylindrical portion such that its tip **354** protrudes through the opening **342** when the cylindrical portion is in the position shown in FIGURE 19. The piston shaft **352** is preferably in general alignment with a solid portion, e.g. a horizontal support bar **356,** of the rack. As shown in FIGURE 12, the point of contact with the rack is spaced from the top of the rack so that the rack pivots around the hooks **210** when the contact point is displaced.

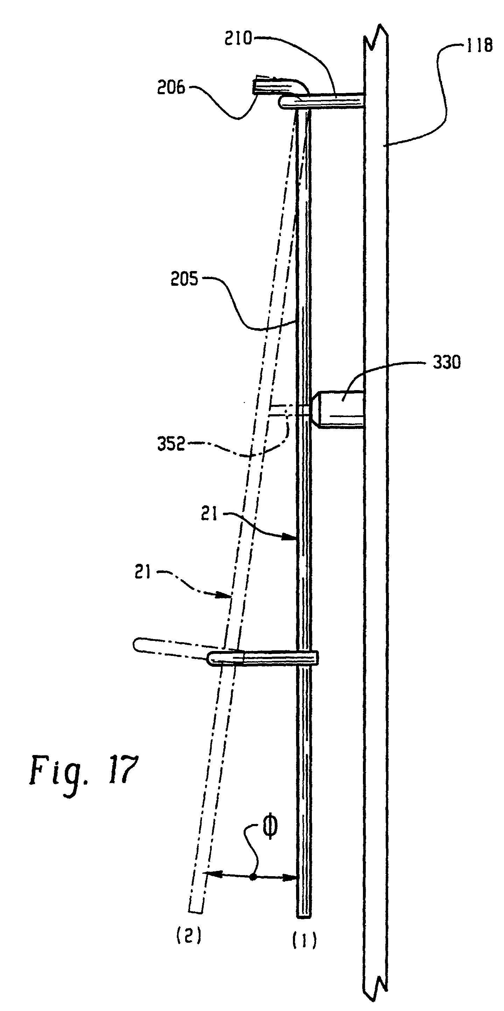

When the rack **21** is stationary, it hangs in a vertical, or substantially vertical, position within the chamber (FIGURE 17, solid lines). When the piston is driven forward to the position shown in FIGURE 19, the piston shaft contacts or strikes the support **356.** Upon striking the support bar **356,** the rack is displaced from its vertical position and pivoted at an angle φ away from vertical to an angled position, (FIGURE 17, hatched lines). The displacement angle φ is dependent on the force with which piston **344** strikes the rack. When the piston is returned to the position shown in FIGURE 18, the rack falls back to its original position and, depending on the force used and speed of extending and retracting the piston, may bounce one or more times on the cylinder before settling back to rest.

Preferably, the piston shaft **352** is forced out of the cylinder with a force sufficient to displace the rack from its resting vertical position and, most preferably, cause the rack to vibrate. The force of the piston should not be excessive such that the any portion of the endoscope becomes permanently dislodged from the rack. Displacement of the rack from its resting vertical position changes the angular position of the rack and the endoscope with respect to the spray jet nozzles, allowing the endoscope to be exposed to different spray contact angles. Vibrating preferably changes the position of the endoscope on the support pegs or any other support member of the rack. Specifically, vibrating the rack results in a change of the position of the endoscope. Changing the position of the endoscope on the support pegs changes the contact site, i.e., the portion of the endoscope making direct contact with the support peg or other portion of the rack. Therefore, upon movement and vibration of the rack, new surface areas of the endoscope are exposed to the spray nozzles, such that all of the endoscope is effectively cleaned. Additionally, displacing and vibrating the rack from its resting position prevents seals between the endoscope and the contact point of the rack from forming.

The piston **344** is driven by a driving system **360,** such as a motor drive system or a pneumatic or hydraulic drive system. In a pneumatic system, a gas supply, such as an air tank, pump, compressor or the like (not shown) supplies air to a rearward portion **362** of the cavity **340,** located rearward of the cylindrical portion of the piston, through a first air inlet line **364.** As the air enters (activation), the tip of the piston shaft **352** is driven forward and outward from the housing. The piston may be returned to its deactivated position by allowing the air to flow out of the rear chamber **362.** More preferably, the piston is withdrawn back into the cylinder, i.e., is deactivated, by forcing air via a second air inlet line 366 into a forward portion **368** of the cavity **340.** During the activation portion of the cycle, air may be displaced from the forward portion **368** of the cavity via line **366** and, similarly, during deactivation, air may be be displaced from the rearward portion **362** of the cavity via line **364.**

For example, air is pulsed into the housing every 1-20 seconds, preferably, about every 10 seconds. Activating the cylinder at regular short intervals may set the rack in "continuous" motion, i.e., the rack is basically constantly moving and does not remain at rest (in the vertical position) for significant periods of time. Studies comparing the pulsed rack with a stationary rack have found a reduction in microbial count as the instruments are decontaminated, particularly when cleaned with relatively low levels of antimicrobial agents.

The housing **338** and piston **344** reside in the chamber **12** and thus are both within the sterile fluid pathway. To avoid harboring and growth of microorganisms within the housing and possible recontamination of the sterilized endoscopes after the sterilization portion of the cycle it is desirable to seal the cavity from the chamber **12.** A sealing member **370,** such as a gasket, is positioned between the housing and the wall and held in place by the screws **346.** A second sealing member **372** is received in a groove **374**, in the opening bore **342.**

The activation system can be activated in any cycle or phase of cleaning and decontamination. Preferably, the system is activated during each of the cleaning, sterilization, and rinsing cycles. Moving the rack during each of the cycles allows for all areas of the endoscope to be effectively rinsed, cleaned, and sterilized.

In a typical decontamination cycle, items to be decontaminated are first inserted into the cabinet **10** through the opening **14,** with the door **18** open, as shown in FIGURE 2. The endoscope **B** to be cleaned is mounted on the rack **21** and inserted into the chamber **12** with other items to be cleaned and decontaminated. The tubes **180** are connected with their respective endoscope inlet ports **186** and connection ports to connect the endoscope internal passages with the fluid lines. The biopsy forceps are loaded on the rack **21**. One or more endoscope valves may be inserted in respective valve reprocessors. The leak detector **204** is connected with the endoscope venting connector port. A fresh cup **44** of concentrated decontaminant and other components is inserted into the well **30** and a restraining member or lid **384** positioned over the cup.

Once all the items are properly positioned and fluid lines connected, the door **18** is brought into the closed position, as shown in FIGURE 13. The latching mechanism **238** is then pivoted around the pivot points **256** until the rollers **264** engage the camming surface **274** as shown in FIGURE 14, indicating that the door is fully closed and fully latched. As the latching mechanism is moved from the disengaged to the engaged position, the geometry of the camming surface, the rollers, and the arm pivot point, along with the spring energy provided by the compression seal, result in the final positioning of the rollers being "over center."

The sensor **310,** mounted on the forward end of the forward end of the latching arm, senses that the latching arm is properly positioned adjacent the camming surface and signals the control system **80** that the latching mechanism is engaged. The control system then signals the air cylinder **284** to move the piston rod **282** from a lowered, or unlocked position to the locked position, such that the piston rod engages the outer side of the latch arm plate **254.** The sensor **312** in the locking mechanism detects that the piston rod is in the locked position, and signals the control system **80** that the latching mechanism is locked in position. The control system does not commence a washing and decontamination cycle until the sensors **310, 312** register that the latching mechanism **238** is properly engaged and that the locking mechanism **280** is in the locked position. At the end of the cycle the control system signals the locking mechanism to retract the locking rod. The latching mechanism can then be withdrawn from engagement with the camming surface.

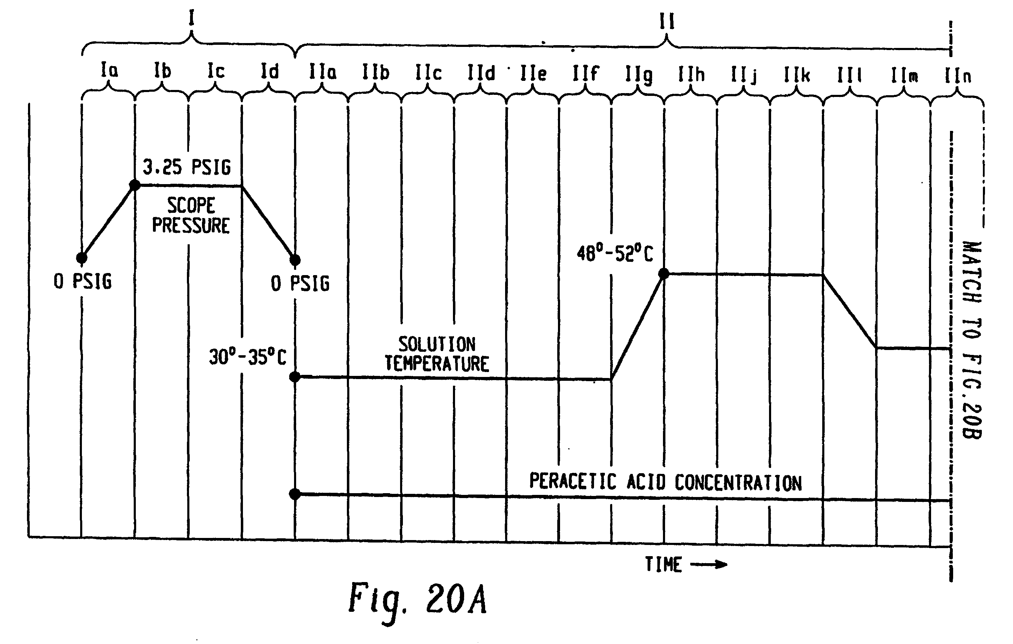

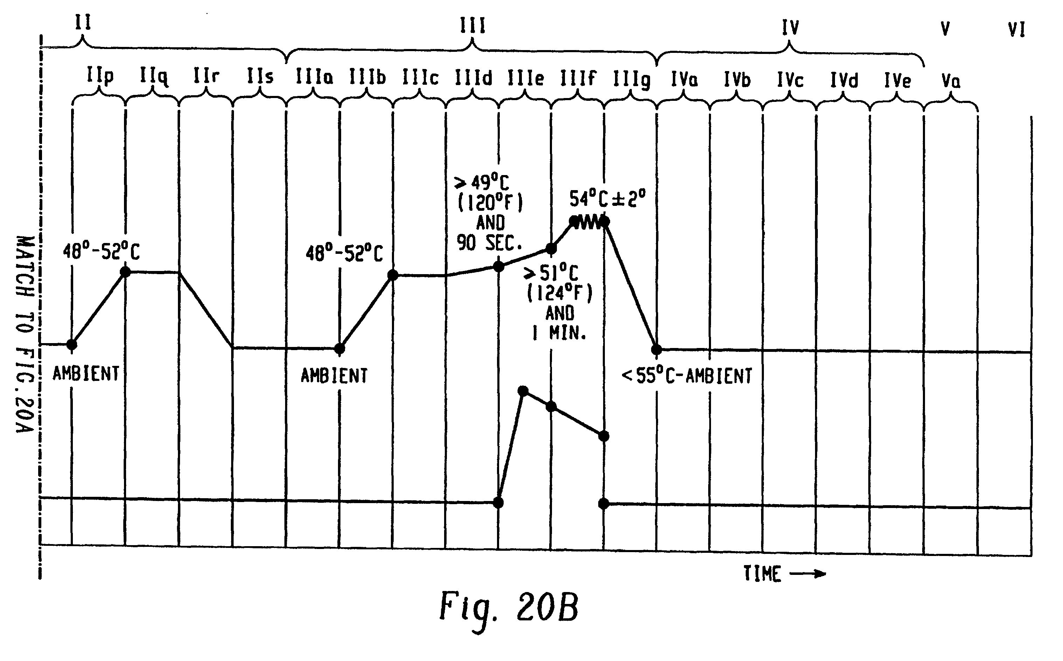

With reference to FIGURE 3 and also to FIGURE 20, the entire process, including door locking, leak testing, washing, microbial decontamination, and rinsing steps, is fully automated. There is no need for an operator to contact the items until all of the steps are complete. As shown in FIGURE 20, a typical cycle includes five phases, a leak testing phase **I**, a prerinse and washing phase **II**, a microbial decontamination phase **III,** a rinse phase **IV,** and a drying phase **V,** which are carried out in sequence.

In phase **I,** the control system **80** signals the leak tester **204** to check the endoscope for leaks. If all is satisfactory, phase II begins. The control system can be programmed to skip this step, if, for example, the device does not have an internal passage to be tested.

In phase II, the items are preferably subjected to a prerinse operation, stages **IIb-IId,** in which the items are sprayed externally and flushed internally with warm (about 30-35°C) water for about one minute to remove the bulk of gross debris. The temperature of the water is selected to prevent protein denaturation. Denatured proteins adhere to surfaces and are difficult to remove. Accordingly, the water is kept below 40°C to prevent this denaturation. All of the soil and other debris which is rinsed off the device is captured in a filter **386,** such as a backwashable drain strainer, and is not recirculated through the fluid distribution system. During drain portions of the cycle, the filter is flushed to remove debris.

After about 1 minute of prerinsing, the control system signals a drain valve **388B** in the fluid line **60** to open and the rinse water is flushed from the system A to the drain. A portion of the rinse and cleaning fluids is backflushed through the backwashable drain strainer and out to the drain through valve **388A** to remove accumulated debris.

As shown in FIGURE 3, the backwashable drain strainer can be isolated from the fluid pathways using valves **389A** and **389B.** This allows the backwashable drain filter to bypassed during the sterilization portion of the cycle. This ensures that debris trapped on the filter is not later released into the fluid pathway which may recontaminate the endoscope.

In stage **IIe,** the endoscope is flushed with air. Specifically, the control system **80** signals a valve **390** in an air line **392** to open and supply microbe-free compressed air to the system to remove excess water from the items. The air is preferably passed through a HEPA microbe removal filter 394 before entering the system.

In stage **IIg**, the computer control **80** signals the valve **52** in the water inlet line **42** to open, allowing water to circulate through the well and the fluid lines **60.** In stage **IIh,** the heater **64** heats the water to a suitable temperature for cleaning. The temperature selected is within the range of temperature to which the device may be subjected, while providing effective cleaning. For endoscopes which have a maximum rating of 60°C, that are being cleaned with a detergent based washing solution, a preferred washing solution temperature is from about 48-52°C. If an enzymatic cleaner is to be used, the temperature selected will also depend on the stability and operating temperatures of the enzymes employed.

In stage **IIj,** the computer control system **80** signals the opening member **48** to open the cleaner compartment **45** of the cup. The cleaner concentrate mixes with the water to form the washing solution and is delivered by the pump **22** under pressure to the nozzles **102, 104, 106, 108, 110** and endoscope connection ports **150, 152, 154, 156** in stage **IIk.** The nozzles spray the washing solution over the outer surfaces of the items while the connection ports deliver the solution to the internal passages, thereby cleaning inner and outer surfaces simultaneously. Sprayed washing solution, which drips off the items, is collected in the sump **22.** The pump **22** returns the collected solution from the sump to the fluid supply line **60,** preferably after first passing at least a part of the collected solution through the well **30** to ensure complete mixing of the cleaner in the solution. A sensor **398,** such as a conductivity detector detects whether there is concentrated cleaner in the washing solution, for example, by measuring the conductivity of the circulating washing solution.

The washing solution removes soil from the items, leaving them clean, but not necessarily free of viable microorganisms. The spray jets are particularly effective in this physical cleaning stage.

If the instruments to be cleaned have been left for a relatively long period between use and processing (greater than about an hour), it is preferable to use an enzymatic soak prior to, or in place of, the washing phase. This helps to loosen the blood and other proteins, which gradually harden and become difficult to remove. The enzymatic soak preferably lasts from about 10 minutes to about an hour. In the soak, the enzymatic washing solution is circulated slowly through the system. An additional compartment may be provided in the cup **44,** if enzymatic cleaning as well as detergent washing steps are to be used. The control system **80** is programmable to provide for an enzymatic soak in place of, or in addition to, a normal washing step.

Once the washing solution has been circulated through the system for sufficient time to remove the soil from the endoscope and other items, the control system signals drain valves **388A** and **388B** in the fluid line **60** to open and the washing solution is flushed from the processor **A** to the drain. Optionally, in stage **II1,** the water inlet valve **52** is opened to allow additional fresh water into the system to flush the washing solution from the fluid lines **60,24** and the well **30.** The drain valves **388A** and **388B** are then closed. Another air flush/drying step is preferably carried out as stage **IIm** to remove excess water from the items. In stage **IIn-s,** an additional hot water rinse and dry is optionally carried out.

Optionally, the devices are manually cleaned, rather than being washed in the processor **A**. In such cases, the operator programs the control system **80** to skip the washing and optionally the rinsing steps **IIj-s.** A cup **44** which lacks the compartment holding the concentrated cleaning agent is used.

In stage **IIIa-c,** the control system **80** opens the valve **52** for a short period to allow more water into the processor and signals the heater to heat the water. Once sufficient water has entered the system for carrying out the decontaminant part of the cycle, the controller **80** signals the valve **52** to close. The control system **80** signals the cup cutter **48** to open the second compartment **46** of the cup **44,** containing the pretreatment components (stage **IIId**). These are released into the fluid lines and are circulated through the processor as a pretreatment solution. The pump **22** circulates the pretreatment solution so that the pretreatment chemicals are distributed throughout the processor **A** and over the items to be microbially decontaminated, prior to admission of the decontaminant. The pretreatment components buffer the water in the fluid lines to an appropriate pH (typically pH 5-9) for effective decontamination. The corrosion inhibitors present coat the parts of the processor to be exposed to the decontaminant solution and the surfaces of items to be decontaminated with traces of inhibitors to provide resistance to the corrosive effects of the decontaminant.

Although the pretreatment components may be alternatively included in one or other of the cleaner and decontaminant compartments **45, 47** their effectiveness is lessened. By releasing corrosion inhibitors before the microbial decontaminant, the inhibitors are assured time to develop protective barriers around the parts before the parts are contacted by the decontaminant. The buffers modify the pH of the fluid circulating in the system to near neutral with a preferred pH of 6-8. Until the buffer has circulated throughout the system, the microbial decontaminant is not fully effective. Additionally, such agents may degrade the microbial decontaminant during storage. Accordingly, it is preferable to provide a separate compartment **46** for the pretreatment components and allow them to circulate through the system for a period of time before introducing the decontaminant.

After a preselected period of circulation, the controller **80** signals the cutter assembly to open the third compartment **47** (stage **IIIe).** The decontaminant then mixes with the pretreatment components in the fluid lines **60, 24** and is sprayed through the nozzles **102, 104, 106, 108, 110** and delivered to the endoscope connection ports **150, 152, 154, 156,** so that the decontaminant solution flows over the exterior surfaces and through the internal passages of the items to be decontaminated (stage **IIIf**). The nozzles pulse the decontaminant fluid in a preselected sequence to ensure full coverage of the spray.

For a short period of time, valve **389A** is left open so that the backwashable drain strainer **386** is sterilized with the decontaminant solution. The decontaminant solution flowing through the backwashable drain strainer is passed to the drain through valve **388A.** Then, valves **389A** and **3898** are closed and the decontaminant solution is all rerouted through a line **400** running parallel to the main fluid line **60** by opening a valve **402** in line **400** (FIG. 3). The remaining decontaminant solution thus bypasses the backwashable drain strainer to avoid recontaminating the endoscope.

A decontaminant sensor **402** in fluid communication with one of the fluid flow lines **60, 24** optionally detects the concentration of the decontaminant in the circulating fluid to ensure that a threshold concentration for effective decontamination is provided. The control system controls the heater so that an optimum temperature for decontamination is maintained. Once again, the optimum temperature is dependant on the maximum rating for the device being decontaminated, and also on the effective temperature for the decontaminant. For peracetic acid sterilization of endoscopes rated to 60°C, a preferred minimum temperature of about 48-55°C, more preferably, about 50°C, for the circulating decontaminant solution is maintained.

The chamber is maintained under a slight positive pressure during decontamination to minimize ingress of outside air into the chamber. Air exits the chamber through vents (not shown), which provide a tortuous pathway to minimize air ingress.

After a period of circulation of the decontaminant solution sufficient to effect decontamination of the items (typically about 10-15 minutes for complete sterilization, more preferably, about 12 minutes; 2-5 minutes for high level disinfection, more preferably, about 3 minutes), the drain valve **388** in the processor **A** is opened and the decontaminant solution flushed from the processor **A** to the drain (stage **IIIg).** The circulation period is optionally adjusted in accordance with monitored decontaminant levels during the cycle.

The rinse phase **IV** then begins. The drain valve **388** is kept open and the control system opens a valve **404** to allow a source **406** of sterile rinse water to supply sterile water to the fluid lines **60** for rinsing the decontaminated items without risk of recontamination of the decontaminated items. The source of sterile water preferably comprises a water heater **408** which heats incoming tap water to a sufficient temperature to destroy microorganisms (preferably about 150 °C) in the water, and a heat exchanger **410,** which transfers excess heat from the sterilized water to the incoming tap water (FIGURE 3). The water heater helps remove salts from the incoming water which could otherwise deposit on the washed and microbially decontaminated instruments. The water produced by the sterile water generator **406** is thus of high purity. The sterile water generator **406** provides water on demand, eliminating the need to store large quantities of sterile water. The rinse phase may include several fill and blow off stages **(IVa-e).**

Alternatively, the water inlet valve **52** is opened once more to provide rinse water for rinsing the decontaminated items again.

The system **A** has a fill of about 9 liters. A typical cycle includes 6 fills, for a total fluid requirement of 54 liters, as follows:
1) for pre-rinsing,
2) for forming the washing solution,
3) rinsing the washing solution from the system,
4) for forming the pretreatment and decontaminant solution, and
5) and 6) for sterile rinsing.

After the rinse water has been discharged to the drain, the control system **80** signals the valve **390** in air line **392** to open and supply microbe-free air to the system to blow accumulated water out of and off of the decontaminated items. The air line is connected with the manifold **26** so that the air flows through the nozzles and connection ports, drying the interior and exterior surfaces of the endoscopes and other items. The regulator valves **168, 170, 172,** and **174** ensure that the internal passages of the endoscope B are not pressurized beyond their recommended pressure ratings.

Optionally, an alcohol flush is used in addition to, or in place of the last of the rinse steps **IV a-e.** In this case, a source of alcohol **420** supplies the alcohol to the chamber to remove excess water from the device **B.** Remaining alcohol quickly evaporates from the device. FIGURE 3 shows the source of alcohol connected with the connection ports **150, 152, 154**, via a pump **422** for delivering the alcohol to the internal lumens of the device, although it is also contemplated that the alcohol may be supplied to the nozzles also, for drying the exterior of the device.

Optionally, the device may be kept in the chamber for an extended period, such as overnight, to increase water removal. Or the air used to flush the device may be heated to increase evaporation and water removal.

At the end of the cycle (stage **VI),** the controller **80** signals the cutter assembly **48** to retract from the cup **44** to its starting position and the door locking mechanism to disengage.

Because the steps of leak testing, washing, decontaminating, rinsing, and air drying are carried out automatically and sequentially within the chamber, the entire reprocessing cycle can be carried out in a relatively short period of time, typically 30 to 40 minutes for full sterilization, 20-30 minutes for high level decontamination. The endoscopes are thus ready for reuse in a much shorter time than conventional cleaning and decontamination processes, in which an operator carries out the reprocessing steps using a number of separate pieces of equipment.

The decontaminated items are removed from the decontamination chamber **12** for immediate use or transferred to sterile pouches and stored until needed. The rack **21** can be used to transport the endoscope **B** to a storage cabinet or to a surgery. The rack handle is configured for carrying the rack and for supporting the rack in the storage cabinet. Thus, the endoscope need not be touched until it is to be used in a surgical procedure, minimizing the potential for contamination.

Optionally, the device **B** is enclosed in a sterile pouch before removal from the chamber, to minimize airborne re-contamination prior to reuse. For example, the device may be wrapped in a bag, within the chamber, prior to opening of the door. This may be achieved by suitable controls, or manually, for example with a glove box-type of device in which the operator removes the various connectors from the device and wraps the device in the bag using sterile gloves (not shown) which extend into the chamber.

In an alternative embodiment, the chamber **12** acts as a sterile pouch and is hermetically sealed and disconnected from the rest of the processor **A** and transported to the site at which the decontaminated items are to be used.

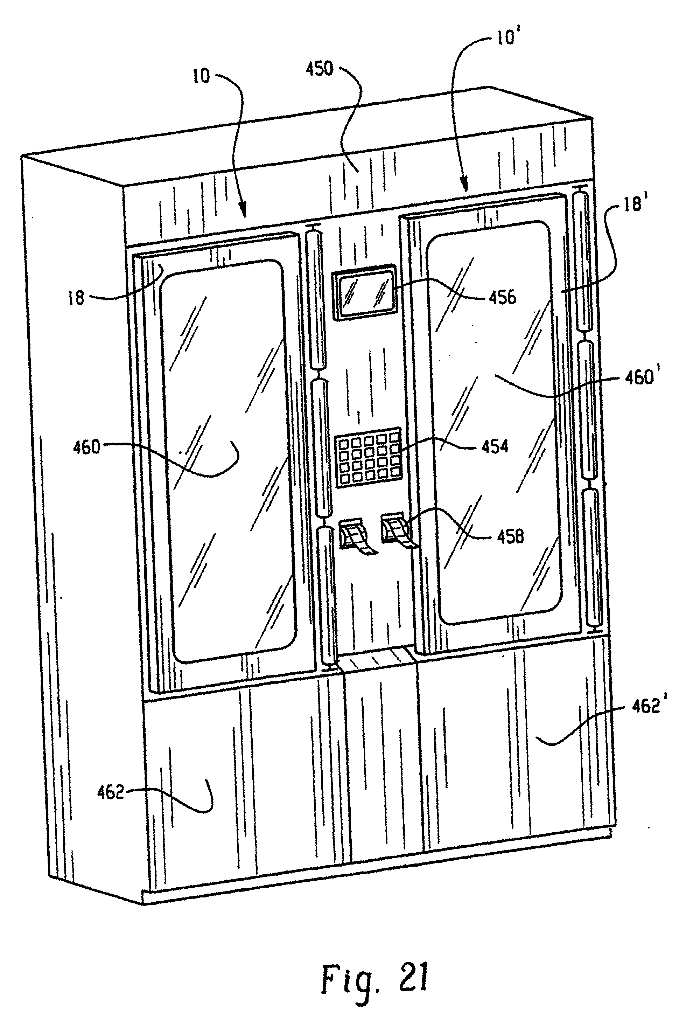

With reference to FIGURE 21, a pair of the cabinets **10, 10'** are mounted side by side in a common frame **450.** A control panel **452** for controlling the cycles in both cabinets is disposed between the two cabinets. The control panel includes a touch input device **454,** such as a touch screen or key pad, which an operator selects among the cycle options and inputs other commands to the control system **80.** The control system controls the cycles in each cabinet independently, allowing cycles to be run asynchronously. An electronic display **456** provides real time information to the operator about the state of the system and cycles in progress. A pair of printers provide printouts **458** descriptive of each sterilization cycle. The printout is available to be transported with the decontaminated endoscope for record keeping purposes. Preferably, an electronic record is also maintained.

The cabinets **10, 10'** each have doors **18, 18'** that include large see-through windows **460, 460'.** These windows enable the operator to monitor the inside chambers of the cabinets during and between cycles. The frame **450** includes door panels **462, 462'** that provide front access to water filters and other service items.

Suitable concentrated cleaning agents are low foaming detergents or enzymatic cleaners, with a pH close to neutral (preferably pH 6-8), to minimize corrosion of metal components.

Various antimicrobial agents may be utilized for the decontaminant. In a preferred embodiment, the decontaminant is a solution of peracetic acid. However, it is also contemplated using other liquid or powdered decontaminants or reagents which react in a common solvent to generate peracetic acid, chlorine, hydrogen peroxide, hypochlorous acid, hypochlorite, or other strong oxidants which have biocidal effects. Aldehydes, such as glutaraldehyde, may be used, but the decontaminant solution should be collected after use and properly treated, rather than disposed of via the drain.

Preferably, the pretreatment agent includes a buffer and a corrosion inhibitor. One preferred buffering system includes a combination of monosodium phosphate, disodium phosphate and hexametaphosphates. Such a buffering system also provides anticorrosion properties. Wetting agents and other corrosion inhibitors may alternatively be used. Preferred copper and brass corrosion inhibitors include azoles, benzoates, other five-membered ring compounds, benzotriazoles, polytriazoles, mercaptobenzothiazole, and the like. Other anti-corrosive compounds include phosphates, molybdates, chromates, dichromates, tungstates, vanadates, borates, and combinations thereof.

The corrosion inhibitory agents are selected in accordance with the nature of the materials in the items being cleaned and/or decontaminated with the decontaminant. Corrosion inhibitors which protect against corrosion of aluminum and steel, including stainless steel, include phosphates, sulfates, chromates, dichromates, borates, molybdates, vanadates, and tungstates. Some additional aluminum corrosion inhibitors include 8-hydroxyquinoline and ortho-phenylphenol.

More specifically, phosphates are preferred for inhibiting stainless steel corrosion. Preferred phosphates include, but are not limited to, monosodium phosphate (MSP), disodium phosphate (DSP), sodium tripolyphosphate (TSP), sodium hexametaphosphate (HMP), and sodium sulfate either alone or in combination. Preferred borates include sodium metaborate (NaBO₂).

Copper and brass corrosion inhibitors include triazoles, azoles, benzoates, tolyltriazoles, dimercaptothiadiazoles, and other five-membered ring compounds. Particularly preferred copper and brass corrosion inhibitors include sodium salts of benzotriazole and tolyltriazole which are preferred due to their stability in the presence of strong oxidizing compounds. Mercaptobenzothiazole can also be utilized but is apt to be oxidized or destabilized by strong oxidizers. Salicylic acid is an example of an acceptable benzoate corrosion inhibitor.

In hard water, phosphate buffers and corrosion inhibitors tend to cause calcium and magnesium salts present in the hard water to precipitate and coat the instruments. being decontaminated and/or cleaned and also leaves deposits on parts of the system. In such cases, a sequestering agent appropriate to prevent precipitation such as sodium hexametaphosphate (HMP), or trisodium nitrolotriacetic acid (NTA Na₃) is preferably provided. Because sodium hexametaphosphate is also a corrosion inhibitor, it serves a dual purpose, both as a corrosion inhibitor and as a sequestering agent. Other sequestering agents include sodium polyacrylates. Of course, if soft or deionized water is utilized, the sequestering agent may be eliminated. However, to ensure universal applicability with any water that might be utilized, the presence of a sequestering agent is preferred.

Surface energy reducing agent (surfactants/wetting agents) are preferably agents to increase penetration into crevices of items being treated. This is particularly important when cleaning and decontaminating complex medical instruments which may contain microbial contaminants in crevices, joints, and lumens. Surface energy reducing agents usable in accordance with the present invention include anionic, cationic, nonionic, amphoteric, and/or zwitterionic surfactants. Specific classes of surfactants which are useful include anionic and nonionic surfactants or combinations thereof. Examples of nonionic surfactants usable in the present invention include surfactants such as fatty alcohol polyglycol ethers, nonylphenoxypoly (ethyleneoxy) ethanol, and ethoxylated polyoxypropylene. Specific examples include Genapol UD-50™, Igepal™, Fluowet™, and Pegal™. The surfactants set forth above may be used alone or in combination with each other.

Amounts of the corrosion inhibitors and surfactants to be used in the peracetic acid solution will vary depending upon the type of agent being added and whether or not one or more agents are added.

The inorganic corrosion inhibitors are preferably present in amounts ranging from about 0.01% to 20.0% weight per volume (w/v). Organic corrosion inhibitors are preferably present in amounts ranging from about 0.01% to 0.5% w/v. Phosphates are effective at concentrations in the range of about 0.01% to about 11.0% w/v.

The surfactants are preferably present in amounts ranging from about 0.0001% to about 5.0% w/v. More preferably, the surfactant is present in amounts ranging from about 0.0001% to about 0.5% w/v.

## Claims

1. An automated system (A) for cleaning and microbially decontaminating a device (B), the automated system including:
a cabinet (10) which defines an interior chamber (12) for receiving the device;
spray nozzles (102, 104, 106, 107, 108, 110), disposed within the chamber, for spraying a washing fluid and a microbial decontaminant fluid over ah external surface (124) of the device;
a source (42, 406) of rinse water;
a source (30, 44, 45) of the washing fluid;
a source (30, 44, 46, 47) of the microbial decontaminant fluid;
a fluid distribution system (26) fluidly connecting the sources of rinse water, washing fluid, and microbial decontaminant fluid with the nozzles;
a pump (24) connected with the fluid distribution system for pumping the washing fluid, microbial decontaminant fluid, and rinse water, to the nozzles and pumps sprayed solutions to the nozzles; and
a control system (80) which controls the delivery of the washing fluid, microbial decontaminant fluid, and rinse water to the nozzles such that the device is sequentially washed with the washing fluid, microbially decontaminated with the microbial decontaminant fluid, and rinsed with the rinse water, the control system (80) sequentially causing a first set (102) of the spray nozzles to spray the decontaminant solution for a first period of time and then causing a second set (104) of the spray nozzles to spray the decontaminant solution for a second period of time.

2. The automated system of claim 1, further **characterized by**:
connection ports (150, 152, 154) , within the chamber (12), connected with the fluid distribution system (26), for connecting with an internal passage (187) of the device (B) such that the internal passage is sequentially washed with the washing fluid, microbially decontaminated with the microbial decontaminant fluid, and rinsed with the rinse water.

3. The automated system of claim 2, further **characterized by**:
a connector **(180,184)** for quickly connecting the connection port **(150, 152, 154)** to the internal passage **(187)** of the device **(B),** the connector being configured to leak a portion of the microbial decontaminant fluid between the connector and an adjacent surface **(186)** of the internal passage of the device to ensure contact of the microbial decontaminant fluid with the adjacent surface.

4. The automated system of any one of preceding claims 1-3, further **characterized by**:
a support member **(21)** for supporting the device within the chamber; and
an activation system **(330)** which agitates the support member to change points of contact between the device **(B)** and the support member.

5. The automated system of claim 4, further **characterized by**:
the activation system **(330)** including a piston **(344)**.

6. The automated system of any one of preceding claims 2-5, further **characterized by**:
the source of rinse water including:
a sterile water generator (406) fluidly connected with the nozzles **(102, 104, 106, 107, 108, 110)** and connection ports **(150, 152, 154),** which sterilizes unsterile water and supplies the sterile water to the nozzles and connection ports for rinsing the device, the control system **(80)** controlling the timing of the delivery of the sterile water.

7. The automated system of any one of preceding claims 1-6, further **characterized by**:
an air line **(322)**, fluidly connected with the fluid distribution system **(26)**, for supplying filtered air to the spray nozzles **(102, 104, 106, 107, 108, 110)** to dry the device.

8. The automated system of any one of preceding claims 1-7, further **characterized by**:
the chamber **(12)** further including a door **(18)** which selectively seals an access opening **(14)** to the chamber, the control system **(80)** preventing opening of the door while the nozzles **(102, 104, 106, 107, 108, 110)** spray the decontaminant fluid over the device **(B).**

9. The automated system of any one of preceding claims 1-8, further **characterized by**:
a latching mechanism **(238),** which selectively moves from a latching position, in which the latching mechanism holds the door **(18)** in a sealing relationship across the access opening **(14),** to an unlatched position, in which the latching mechanism allows opening of the door; and
a locking mechanism **(280),** which selectively allows unlatching of the latching mechanism in an unlocked position and locks the latching mechanism in a locked position, preventing opening of the door, the control system **(80)** controlling movement of the locking mechanism between the unlocked and locked positions.

10. The automated system of claim 9, further **characterized by**:
the latching mechanism **(238)** including:
an arm **(240, 242, 244, 246)** pivotally connected adjacent a first end **(256)** thereof to the cabinet **(10)**, and
a roller **(264)**, rotatably connected to the arm and spaced from the first end, which engages an engagement member **(272)** on an outer surface of the door in the latched position.

11. The automated system of claim 10, further **characterized by**:
the locking mechanism **(280)** further including:
a rod **(282)** which engages the arm **(242)** in the locked position, preventing pivoting of the arm, and allows pivoting of the arm away from the engagement member in the unlocked position, and
a driving member **(284),** the driving member driving the rod between the unlocked position and the locked position.

12. The automated system of claim 11, further **characterized by**:
the driving member **(284)** including an air cylinder.

13. The automated system of any one of preceding claims 1-12, further **characterized by**:
a leak detector connection port **(202)** within the chamber **(12)**, the leak detector connection port adapted for connection with an internal passage of the device; and
a leak detector **(204),** fluidly connected with the connection port, which pressurizes the internal passage with a gas for detecting leaks between a sheath and lumens of the device, the control system **(80)** selectively controlling the operation of the leak detector.

14. The automated system of any one of preceding claims 1-13, further **characterized by**:
at least one of:
a sensor **(398)** which detects the presence of a cleaner in the washing fluid, and
a sensor **(402)** which detects the presence of a decontaminant in the decontaminant fluid.

15. The automated system of any one of preceding claims 1-14, further **characterized by**:
a pair of cabinets **(10, 10')** mounted adjacent each other, each cabinet defining an interior chamber **(12, 12').**

16. The automated system of claim 15, further **characterized by**:
the control system **(80)** controlling the second chamber **(12')** independently of the first chamber **(12)**.

17. The automated system of any one of preceding claims 1-16, further **characterized by**:
the nozzles **(102, 104, 106, 107, 108, 110)** and the device **(B)** being positioned within the chamber **(12)** such that the external surface **(124)** of the device is impacted with a spray **(122)** from at least one nozzle at an angle (θ) of no more than 45 degrees from perpendicular to the external surface.

18. The automated system of claim 17, further **characterized by**:
the nozzles **(102, 104, 106, 107, 108, 110)** and the device **(B)** being positioned within the chamber **(12)** such that every external surface of the device is no more than 20 centimeters from at least one nozzle.

19. The automated system of any one of preceding claims 1-18, further **characterized by**:
at least a first **(102, 104, 106, 107, 110)** of the spray nozzles being positioned on a wall **(114, 116, 118)** of the chamber **(12)** and at least a second **(108)** of the spray nozzles being positioned on a door **(18)** of the chamber.

20. The automated system of any one of preceding claims 1-19, further **characterized by**:
the control system controlling delivery to the nozzles **(102, 104, 106, 107, 108, 110)** such that nozzles whose spray patterns cross and would interfere with each other are operated alternately.

21. The automated system of any one of preceding claims 1-20, further **characterized by**:
The nozzles including a nozzle assembly **(110)** disposed adjacent a geometric center of the chamber **(10)** that sprays radially outward.

22. The automated system of claim 21, further **characterized by**:
the nozzle assembly **(110)** including at least one of:
a plurality of stationary spray heads **(121)** mounted on a central support and directed radially outward, and
a rotating nozzle.

23. The automated system of any one of preceding claims 1-22, further **characterized by**:
a removable rack **(21),** supported within the chamber, for positioning the device such that decontaminant solution sprayed from the nozzles **(102, 104, 106, 107, 108, 110)** contacts all of the external surface **(124)** of the device.

24. The automated system of claim 23, further **characterized by**:
the rack **(21)** including members **(212)** which position the device such that flexible portions of the device are bent into an arc with a minimum radius of no less than 15 centimeters.

25. The automated system of either claim 23 or claim 24, further **characterized by**:
the rack **(21)** including pegs which hold the difficult to clean portions of the device in reproducible positions to receive optimal spray from the nozzles.

26. The automated system of either one of preceding claims 24 and 25, further **characterized by**:
a clip **(214, 214')** which releasably couples a tip portion of the device **(B)** to one of the rack **(21)** and another portion of the device, to keep the tip portion securely positioned.

27. The automated system of claim 26, further **characterized by**:
the clip **(214, 214')** including at least one upper finger **(218, 218')** and at least one. lower finger **(220, 220'),** the fingers each contacting the device at a ridge **(222)** to minimize contact between the clip and the device.

28. The automated system of claim 27, further **characterized by**:
the clip **(214)** being adapted for interconnection with the fluid distribution system **(26)** and including:
apertures **(224)** defined in the fingers **(218, 218', 220, 220')** for delivering decontaminant solution to portions of the endoscope contacted by the fingers.

29. The automated system of any one of preceding claims 1-28, further **characterized by**:
the fluid distribution system **(26)** including:
a first fluid pathway **(160, 162)** connected with a first connection port **(150A, 150B),**
a first regulator **(168, 170)** regulating the pressure of the decontaminant solution flowing through the first fluid pathway to a first internal passage of the device,
a second fluid pathway **(164)** connected with at least a second connection port **(152),** and
a second regulator **(172)** regulating the pressure of the decontaminant solution flowing through the second fluid pathway to a second internal passage of the device, such that maximum pressure ratings of the first and second internal passages are not exceeded.

30. The automated system of any one of preceding claims 1-29, further **characterized by**:
a valve reprocessor **(158)** connected with the fluid distribution system **(26)**, for receiving an endoscope valve **(234)**, the valve reprocessor opening the valve while flowing the solutions over and through the valve.

31. The automated system of any one of preceding claims 1-30, further **characterized by**:
a backwashable drain filter **(386)**, connected with the fluid distribution system **(26)** which removes gross debris from the circulating fluids.

32. A method of cleaning and microbially decontaminating a device **(B)** comprising the sequential steps of:
(a) positioning the device within a chamber **(12)**;
(b) spraying a washing solution over the device from nozzles **(102, 104, 106, 107, 108, 110)** within the chamber to remove soil from exterior surfaces **(124)** of the device;
(c) spraying a microbial decontaminant solution over the device from nozzles **(102, 104, 106, 107, 108, 110)** within the chamber to microbially decontaminate the exterior surfaces of the device, including:
alternately spraying with different groups of nozzles, a first set **(102)** of the spray nozzles spraying the decontaminant solution for a first period of time and then a second set (104) of the spray nozzles spraying the decontaminant solution for a second period of time; and
(d) spraying a rinse fluid over the device from nozzles **(102, 104, 106, 107, 108, 110)** within the chamber to rinse the exterior surfaces of the device.

33. The method of claim 32, further **characterized by**:
at least one of:
step (b) further including circulating the washing solution through a lumen **(187)** of the device,
step (c) further including circulating the microbial decontaminant solution through the lumen of the device, and
step (d) further including circulating the rinse fluid through the lumen of the device.

34. The method of claim 33, further **characterized by**:
supplying the washing or decontaminant solution to the lumen through a leaking connector **(184)** which allows a portion of the solutions to leak from the device around the connector.

35. The method of either one of preceding claims 33-34, further **characterized by**:
step (d) including:
sterilizing water to form the rinse fluid.

36. The method of any one of preceding claims 33-35, further **characterized by**:
(e) spraying the endoscope exterior with alcohol and circulating alcohol through the lumen, and
draining the alcohol.

37. The method of any one of preceding claims 33-36, further **characterized by**:
(e') after step (d) blowing filtered air through the lumen.

38. The method of any one of preceding claims 32-37, further **characterized by**:
between steps (a) and (b):
leak testing at least one internal passage of the device, continuing with step (b) only in response to passing the leak test.

39. The method of any one of preceding claims 32-37, further **characterized by**:
supporting the device on a support member **(21)**; and
agitating the support member to change points of contact between the device **(B)** and the support member.

40. The method of claim 39, further **characterized by**:
the step of agitating the support member including:
striking the support member at intervals such that the support member pivots around a pivot point **(210)**.

41. The method of any one of preceding claims 32-40, further **characterized by**:
prior to step (b):
spraying a rinse liquid over the device from nozzles within the chamber at a temperature below about **35°C** to remove a portion of the soil without substantial denaturation of proteins in the soil.

42. The method of any one of preceding claims 32-41 further **characterized by**:
between steps (b) and (c):
spraying a pretreatment solution over the device which includes at least one of a buffering agent and a corrosion inhibitor.

43. The method of claim 42, further **characterized by**: the pretreatment solution including a buffering agent, a corrosion inhibitor, and a surfactant.

44. The method of either one of preceding claims 42 and 43, further **characterized by**:
after the step of spraying the pretreatment solution and prior to step (c):
combining the pretreatment.solution with an antimicrobial decontaminant to form the decontaminant solution.

45. The method of any one of preceding claims 32-44, further **characterized by**:
mounting a second device in a second chamber;
with a common controller, causing steps (b), (c), and (d) to be performed independently and asynchronously in the two chambers.

46. The method of any one of preceding claims 32-45, further **characterized by**:
the decontaminant solution including peracetic acid.

47. The method of claim 46, further **characterized by**: during step (c):
monitoring the concentration of peracetic acid in the decontaminant solution.

48. The method of claim 47, further **characterized by**:
if the concentration of peracetic acid falls below a predetermined level; performing at least one of:
terminating the method,
signaling that the peracetic acid is below the predetermined level,
adding additional peracetic acid to the decontaminant solution, and
extending step (c) for a sufficient period of time to microbially decontaminate the exterior surfaces.

49. The method of any one of preceding claims 32-48, further **characterized by**:
step (a) including:
loading the device into the chamber through an opening **(14)**;
and the method further including:
sealing the opening with a door **(18)**,
moving a latching mechanism **(238)** from an unlatched position, in which the latching mechanism allows opening of the door, to a latching position, in which the latching mechanism holds the door in a sealing relationship across the access opening, and
moving a locking mechanism **(280)** from an unlocked position, in which the locking mechanism allows unlatching of the latching mechanism to a locked position, in which the locking mechanism locks the latching mechanism, preventing opening of the door.

50. The method of claim 49, further **characterized by**:
after the step of moving the latching mechanism to the latching position:
automatically sensing that the door is in the latching position before moving the locking mechanism to the locked position.

51. The method of claim 50, further **characterized by**:
controlling the locking mechanism during steps (b)-(d) to ensure that the locking mechanism does not move to the unlocked position until step (d) is complete.

52. The method of any one of preceding claims 49-51, further **characterized by**:
the step of moving the latching mechanism to the latching position including:
pivoting at least one arm **(240, 242, 244, 246)** connected with an exterior of the chamber until a roller **(264)**, rotatably connected to the arm, engages an engagement member **(272)** on an outer surface of the door.

53. The method of claim 52, further **characterized by**:
the step of moving the locking mechanism to the locked position further including:
engaging the arm to prevent pivoting of the arm in the locked position.

54. The method of any one of preceding claims 49-53, further **characterized by**:
automatically sensing that the locking mechanism is in the locked position prior to commencing step (b).

55. The method of any one of preceding claims 32-54, further **characterized by**:
step a) including:
. positioning the device within the chamber such that spray jets of the washing solution and the'microbial decontaminant solution from the nozzles impinge on exterior surfaces of the device during steps b)and d), each of the exterior surfaces of the device being contacted with at least one spray jet at an angle of no more than 45 degrees from perpendicular to the device.

56. The method of claim 55, further **characterized by**: step a) further including:
positioning the device within the chamber such that each of the external surfaces of the device is no more than 20 centimeters from at least one of the nozzles.

57. The method of claim 56, further **characterized by**:
the step of positioning the device within the chamber including coiling the device on a rack **(21)**.

58. The method of claim 57, further **characterized by**: after a microbial decontamination cycle:
lifting the rack from the chamber to remove the rack and the device as a unit.

59. The method of any one of preceding claims 32-58, further **characterized by**:
steps c) and e) further including:
supplying the washing or decontaminant solution to a first lumen at a first fluid pressure, and .
supplying the washing or decontaminant solution to a second lumen at a second fluid. pressure.

60. The method of any one of preceding claims 32-59 further **characterized by**:
passing the rinse fluid through a backwashable drain filter to remove gross debris;
passing a portion of the decontaminant solution through the backwashable drain filter to decontaminate the backwashable drain filter.

61. The method of claim 60, further **characterized by**:
isolating the backwashable drain filter from the fluid pathway; and
passing a remaining portion of the decontaminant solution through the fluid pathway.

## Patentansprüche

1. Automatisches System (A) zum Reinigen und mikrobiellen Dekontaminieren eines Geräts (B), wobei das automatische System aufweist:
ein Gehäuse (10), welches eine innere Kammer (12) zur Aufnahme des Geräts begrenzt;
Sprühdüsen (102, 104, 106, 107, 108, 110), die in der Kammer angeordnet sind zum Versprühen einer Waschflüssigkeit und einer mikrobiellen dekontaminierenden Flüssigkeit über eine Außenfläche (124) des Geräts;
eine Quelle (42, 406) für Spülwasser;
eine Quelle (30, 44, 45) für die Waschflüssigkeit;
eine Quelle (30, 44, 46, 47) für die mikrobiellen dekontaminierenden Flüssigkeit;
ein Flüssigkeitsverteilungssystem (26), welches eine Fluidverbindung der Quellen für Spülwasser, Waschflüssigkeit und mikrobielle dekontaminierende Flüssigkeit mit den Düsen schafft;
eine Pumpe (24), die mit dem Flüssigkeitsverteilungssystem zum Pumpen der Waschflüssigkeit, der mikrobiellen dekontaminierenden Flüssigkeit, und des Spülwassers zu den Düsen und die Sprühlösungen zu den Düsen pumpt; und
ein Steuersystem (80), welches die Abgabe von Waschflüssigkeit, mikrobiell dekontaminierender Flüssigkeit und Spülwasser an die Düsen steuert, derart, dass das Gerät der Reihe nach gewaschen wird mit der Waschflüssigkeit, mikrobiell dekontaminiert wird mit der mikrobiellen dekontaminierenden Flüssigkeit und gespült wird mit dem Spülwasser, wobei das Steuersystem (80) der Reihe nach einen ersten Satz (102) von Sprühdüsen die dekontaminierende Flüssigkeit für eine erste Zeitdauer versprühen lässt und dann einen zweiten Satz (104) von Sprühdüsen die dekontaminierende Flüssigkeit für eine zweite Zeitdauer versprühen lässt.

2. Automatisches System nach Anspruch 1, weiterhin **gekennzeichnet durch**:
Verbindungsanschlüsse (150, 152, 154) innerhalb der Kammer (12), die mit dem Flüssigkeitsverteilungssystem (26) verbunden sind zur Verbindung mit einem innen liegenden Durchlass (187) des Geräts (B) derart, dass der innen liegende Durchlass der Reihe nach gewaschen wird mit der Waschflüssigkeit, mikrobiell dekontaminiert wird mit der mikrobiellen dekontaminierenden Flüssigkeit und gespült wird mit dem Spülwasser.

3. Automatisches System nach Anspruch 2, weiterhin **gekennzeichnet durch**:
ein Anschlussstück (180, 184) zur schnellen Verbindung des Verbindungsanschlusses (150, 152, 154) mit dem innen liegenden Durchlass (187) des Geräts (B),
wobei das Anschlussstück dazu konfiguriert ist, eine Menge an mikrobieller dekontaminierender Flüssigkeit zwischen dem Anschlussstück und einer angrenzenden Fläche (186) des innen liegenden Durchlasses des Geräts durchzulassen zur Sicherstellung des Kontakts der mikrobiellen dekontaminierenden Flüssigkeit mit der angrenzenden Fläche.

4. Automatisches System nach einem der vorstehenden Ansprüche 1 - 3, weiterhin **gekennzeichnet durch**:
ein Tragbauteil (21) zum Abstützen des Geräts innerhalb der Kammer; und
ein Aktivierungssystem (330), welches das Tragbauteil zur Veränderung von Kontaktpunkten zwischen dem Gerät (B) und dem Tragbauteil hin- und herbewegt.

5. Automatisches System nach Anspruch 4, weiterhin **dadurch gekennzeichnet, dass**:
das Aktivierungssystem (330) einen Kolben (344) aufweist.

6. Automatisches System nach einem der vorstehenden Ansprüche 2 - 5, weiterhin **dadurch gekennzeichnet dass**:
die Quelle für Spülwasser aufweist:
einen Generator (406) für steriles Wasser, der in Flüssigkeitsverbindung steht mit den Düsen (102, 104, 106, 107, 108, 110) und den Verbindungsanschlüssen (150, 152, 154), der unsteriles Wasser sterilisiert und das sterile Wasser an die Düsen und die Verbindungsanschlüsse speist zum Spülen des Geräts, wobei das Steuersystem (80) die Zeit der Abgabe des sterilen Wassers steuert.

7. Automatisches System nach einem der vorstehenden Ansprüche 1 - 6, weiterhin **gekennzeichnet durch**:
eine Luftleitung (322), die mit dem Flüssigkeitsverteilungssystem (26) in Fluidverbindung steht zur Zufuhr von gefilterter Luft an die Sprühdüsen (102, 104, 106, 107, 108, 110) zum Trocknen des Geräts.

8. Automatisches System nach einem der vorstehenden Ansprüche 1 - 7, weiterhin **dadurch gekennzeichnet dass**:
die Kammer (12) eine Türe (18) aufweist, die wählbar eine Zugangsöffnung (14) zur Kammer abdichtet, wobei das Steuersystem (80) ein Öffnen der Türe verhindert, während die Düsen (102, 104, 106, 107, 108, 110) die dekontaminierende Flüssigkeit über dem Gerät (B) versprühen.

9. Automatisches System nach einem der vorstehenden Ansprüche 1 - 8, weiterhin **gekennzeichnet durch**:
einen Klinkenmechanismus (238), der sich selektiv von einer Verriegelungsstellung, in der der Klinkenmechanismus die Türe (18) in einer abdichtenden Beziehung über der Zugangsöffnung (14) hält und einer unverriegelten Stellung bewegt, in der der Klinkenmechanismus ein Öffnen der Türe gestattet; und
einen Verriegelungsmechanismus (280), der selektiv ein Entriegeln des Klinkenmechanismus zu einer unverriegelten Stellung gestattet und den Klinkenmechanismus in einer verriegelten Stellung verriegelt, die ein Öffnen der Türe verhindert und wobei das Steuersystem (80) die Bewegung des Verriegelungsmechanismus zwischen der entriegelten und verriegelten Stellung steuert.

10. Automatisches System nach Anspruch 9, weiterhin **dadurch gekennzeichnet, dass**:
der Klinkenmechanismus (238) aufweist:
einen Arm (240, 242, 244, 246), der benachbart zu einem ersten Ende (256) davon mit dem Gehäuse (10) gelenkig verbunden ist, und
eine Rolle (264), die drehbar mit dem Arm verbunden ist und vom ersten Ende beabstandet ist, die mit einem Eingriffsbauteil (272) an einer Außenfläche der Türe in der verriegelten Stellung in Eingriff steht.

11. Automatisches System nach Anspruch 10, weiterhin **dadurch gekennzeichnet, dass**:
der Verriegelungsmechanismus (280) weiterhin aufweist:
eine Stange (282), die sich in der verriegelten Position mit dem Arm (242) in Eingriff befindet und ein Verschwenken des Arms verhindert und in der entriegelten Stellung ein Verschwenken des Arms von dem Eingriffsbauteil weg gestattet, und
ein Antriebsbauteil (284), wobei das Antriebsbauteil die Stange zwischen der entriegelten Stellung und der verriegelten Stellung betätigt.

12. Automatisches System nach Anspruch 11, weiterhin **dadurch gekennzeichnet, dass**:
das Antriebsbauteil (284) einen Luftzylinder aufweist.

13. Automatisches System nach einem der vorstehenden Ansprüche 1 - 12, weiterhin **gekennzeichnet durch**:
ein Leckdetektoranschlussstück (202) innerhalb der Kammer (12), wobei das Leckdetektoranschlussstück zur Verbindung mit dem innen liegenden Durchlass des Geräts ausgebildet ist; und
einen Leckdetektor (204), der mit dem Anschlussstück in Fluidverbindung steht, welcher den innen liegenden Durchlass mit einem Gas unter Druck setzt zur Erkennung von Leckagen zwischen einer Umhüllung und Lumen des Geräts, wobei das Steuersystem (80) selektiv die Funktion des Leckdetektors steuert.

14. Automatisches System nach einem der vorstehenden Ansprüche 1 - 13, weiterhin **gekennzeichnet durch**:
wenigstens eines aus einer Gruppe eines:
Sensors (398), der die Gegenwart eines Reinigungsmittels in der Waschflüssigkeit erfasst, und
Sensors (402), der die Gegenwart eines dekontaminierenden Mittels in der dekontaminierenden Flüssigkeit erkennt.

15. Automatisches System nach einem der vorstehenden Ansprüche 1 - 14, weiterhin **gekennzeichnet durch**:
einem Paar an Gehäusen (10, 10'), die benachbart zueinander angeordnet sind, wobei jedes Gehäuse eine innere Kammer (12, 12') begrenzt.

16. Automatisches System nach Anspruch 15, weiterhin **dadurch gekennzeichnet, dass**:
das Steuersystem (80) die zweite Kammer (12') unabhängig von der ersten Kammer (12) steuert.

17. Automatisches System nach einem der vorstehenden Ansprüche 1 - 16, weiterhin **dadurch gekennzeichnet; dass**:
die Düsen (102, 104, 106, 107, 108, 110) und das Gerät (B) so innerhalb der Kammer (12) angeordnet sind, dass die Außenfläche (124) des Geräts von einem Sprühnebel (122) getroffen wird aus wenigstens einer Düse unter einem Winkel (Θ) von nicht mehr als 45° aus einer Senkrechten auf die Außenfläche.

18. Automatisches System nach Anspruch 17, weiterhin **dadurch gekennzeichnet, dass**:
die Düsen (102, 104, 106, 107, 108, 110) und das Gerät (B) so innerhalb der Kammer (12) angeordnet sind, dass jede Außenfläche des Geräts nicht mehr als 20 cm von wenigstens einer Düse beabstandet ist.

19. Automatisches System nach einem der vorstehenden Ansprüche 1 - 18, weiterhin **dadurch gekennzeichnet, dass**:
wenigstens eine erste (102, 104, 106, 107, 110) der Sprühdüsen an einer Wand (114, 116, 118) der Kammer angeordnet ist und wenigstens eine zweite (108) der Sprühdüsen an einer Türe (18) der Kammer angeordnet ist.

20. Automatisches System nach einem der vorstehenden Ansprüche 1 - 19, weiterhin **dadurch gekennzeichnet, dass**:
das Steuersystem die Abgabe an die Düsen (102, 104, 106, 107, 108, 110) derart steuert, dass Düsen, deren Sprühnebelmuster einander kreuzen und überqueren würden, alternierend betrieben werden.

21. Automatisches System nach einem der vorstehenden Ansprüche 1 - 20, weiterhin **dadurch gekennzeichnet, dass**:
die Düsen einen Düsenaufbau (110) benachbart zu einer geometrischen Mitte der Kammer (10) aufweisen, der radial nach außen gerichtet absprüht.

22. Automatisches System nach Anspruch 21, weiterhin **dadurch gekennzeichnet, dass**:
der Düsenaufbau (110) wenigstens eine aufweist aus einer Gruppe von:
einer Vielzahl von stationären Sprühköpfen (121), die an einer mittigen Aufnahme angeordnet sind und radial nach außen gerichtet verlaufen, und
einer rotierenden Düse.

23. Automatisches System nach einem der vorstehenden Ansprüche 1 - 22, weiterhin **gekennzeichnet durch**:
ein abnehmbares Gestell (21), welches innerhalb der Kammer angeordnet ist zur Positionierung des Geräts derart, dass die von den Düsen (102, 104, 106, 107, 108, 110) versprühte dekontaminierende Lösung mit allen Außenflächen (124) des Geräts in Kontakt kommt.

24. Automatisches System nach Anspruch 23, weiterhin **dadurch gekennzeichnet, dass**:
das Gestell (21) Elemente (212) aufweist, die das Gerät derart positionieren, dass flexible Abschnitte des Geräts zu einem Bogen gekrümmt werden mit einem minimalen Radius von nicht weniger als 15 cm.

25. Automatisches System nach entweder Anspruch 23 oder Anspruch 24, weiterhin **dadurch gekennzeichnet, dass**:
das Gestell (21) Stifte aufweist, die die schwierig zu reinigenden Bereiche des Geräts in reproduzierbaren Stellungen halten, um einen optimalen Sprühnebel aus den Düsen aufzunehmen.

26. Automatisches System nach einem der vorstehenden Ansprüche 24 und 25, weiterhin **gekennzeichnet durch**:
eine Haltevorrichtung (214, 214') die lösbar einen Abschnitt des Geräts (B) mit einer Spitze an das Gestell (21) und einen anderen Abschnitt des Geräts knüpft, um den Abschnitt mit der Spitze sicher festzulegen.

27. Automatisches System nach Anspruch 26, weiterhin **dadurch gekennzeichnet, dass**:
die Haltevorrichtung (214, 214') wenigstens einen oberen Finger (218, 218') und wenigstens einen unteren Finger (220, 220') aufweist, wobei die Finger jeweils das Gerät an einem Rand (222) kontakten zur Minimierung des Kontakts zwischen der Haltevorrichtung und dem Gerät.

28. Automatisches System nach Anspruch 27, weiterhin **dadurch gekennzeichnet, dass**:
die Haltevorrichtung (214) zur Verbindung mit dem Flüssigkeitsverteilungssystem (26) ausgebildet ist und aufweist:
Öffnungen (224), die in den Fingern (218, 218', 220, 220') ausgebildet sind zur Abgabe von dekontaminierender Lösung an von den Fingern kontaktierten Abschnitten des Endoskops.

29. Automatisches System nach einem der vorstehenden Ansprüche 1 - 28, weiterhin **dadurch gekennzeichnet, dass** das Flüssigkeitsverteilungssystem (26) aufweist:
eine erste Flüssigkeitsbahn (160, 162), die mit einem ersten Verbindungsanschluss (150A, 150B) verbunden ist,
einen ersten Regler (168, 170), der den Druck der dekontaminierenden Lösung regelt, die durch die erste Flüssigkeitsbahn zu einem ersten innen liegenden Durchlass des Geräts strömt,
eine zweite Flüssigkeitsbahn (164), die mit wenigstens einem zweiten Verbindungsanschluss (152) verbunden ist, und
einen zweiten Regler (172), der den Druck der dekontaminierenden Lösung regelt, die durch die zweite Flüssigkeitsbahn zu einem zweiten innen liegenden Durchlass des Geräts strömt derart, dass die maximalen Druckwerte des ersten und zweiten innen liegenden Durchlasses nicht überschritten werden.

30. Automatisches System nach einem der vorstehenden Ansprüche 1 - 29, weiterhin **gekennzeichnet durch**:
eine Vorrichtung (158) zur Wiederaufarbeitung eines Ventils, die mit dem Flüssigkeitsverteilungssystem (26) verbunden ist zur Aufnahme eines Endoskopventils (234), wobei die Vorrichtung zur Wiederaufarbeitung eines Ventils das Ventil öffnet, während die Lösungen über und **durch** das Ventil fließen.

31. Automatisches System nach einem der vorstehenden Ansprüche 1 - 30, weiterhin **gekennzeichnet durch**:
einen rückwaschbaren Abzugsfilter (386), der mit dem Flüssigkeitsverteilungssystem (26) verbunden ist und der Schmutz aus den zirkulierenden Flüssigkeiten entfernt.

32. Verfahren der Reinigung und mikrobiellen Dekontaminierung eines Geräts (B), mit den nachfolgenden Schritten:
(a) Positionieren des Geräts innerhalb einer Kammer (12);
(b) Versprühen einer Waschlösung über dem Gerät aus Düsen (102, 104, 106, 107, 108, 110) innerhalb der Kammer zum Entfernen von Ablagerungen von Außenflächen (124) des Geräts;
(c) Versprühen einer mikrobiellen dekontaminierenden Lösung über das Gerät aus Düsen (102, 104, 106, 107, 108, 110) innerhalb der Kammer zum mikrobiellen Dekontaminieren der Außenflächen des Geräts, mit:
alternierendem Besprühen mit unterschiedlichen Gruppen an Düsen,
wobei ein erster Satz (102) der Sprühdüsen die dekontaminierende Lösung während einer ersten Zeitdauer versprüht und dann ein zweiter Satz (104) der Sprühdüsen die dekontaminierende Lösung während einer zweiten Zeitdauer versprüht; und
(d) Versprühen einer Spülflüssigkeit über dem Gerät aus Düsen (102, 104, 106, 107, 108, 110) innerhalb der Kammer zum Spülen der Außenflächen des Geräts.

33. Verfahren nach Anspruch (32) weiterhin **gekennzeichnet durch**:
wenigstens eines Schrittes aus:
Schritt (b), der das Zirkulieren der Waschlösung **durch** ein Lumen (187) des Geräts umfasst,
Schritt (c), der das Zirkulieren der mikrobiellen dekontaminierenden Lösung **durch** das Lumen des Geräts umfasst und
Schritt (d), der das Zirkulieren der Spülflüssigkeit **durch** das Lumen des Geräts umfasst.

34. Verfahren nach Anspruch 33, weiterhin **gekennzeichnet durch**:
Zuführen der Wasch- oder dekontaminierenden Lösung zum Lumen **durch** einen Durchlassanschluss (184), der es einer kleinen Menge der Lösungen gestattet, aus dem Gerät um den Anschluss herum auszutreten.

35. Verfahren nach einem der vorstehenden Ansprüche 33 - 34, weiterhin **gekennzeichnet durch**:
einen Schritt (d) der umfasst:
das Sterilisieren von Wasser zur Bildung der Spülflüssigkeit.

36. Verfahren nach einem der vorstehenden Ansprüche 33 - 35, weiterhin **gekennzeichnet durch**:
(e) Besprühen des Endoskopäußeren mit Alkohol und Zirkulieren des Alkohols **durch** das Lumen, und:
Ablassen des Alkohols.

37. Verfahren nach einem der vorstehenden Ansprüche 33 - 36, weiterhin **gekennzeichnet durch**:
(e') nach dem Schritt (d) Durchblasen von gefilterter Luft **durch** das Lumen.

38. Verfahren nach einem der vorstehenden Ansprüche 32 - 37, weiterhin **gekennzeichnet durch**:
zwischen den Schritten (a) und (b):
Testen wenigstens eines innen liegenden Durchlasses der Geräts auf ein Leck hin, gefolgt vom Schritt (b) nur in Abhängigkeit vom Bestehen des Lecktests.

39. Verfahren nach einem der vorstehenden Ansprüche 32 - 37, weiterhin **gekennzeichnet durch**:
Anbringen des Geräts an einem Tragbauteil (21); und
Betätigen des Tragbauteils zur Veränderung von Kontaktpunkten zwischen dem Gerät (B) und dem Tragbauteil.

40. Verfahren nach Anspruch 39, weiterhin **dadurch gekennzeichnet, dass**:
der Schritt der Betätigung des Tragbauteils aufweist:
Anstoßen des Tragbauteils in Intervallen derart, dass sich das Tragbauteil um einen Schwenkpunkt (210) herum verschwenkt.

41. Verfahren nach einem der vorstehenden Ansprüche 32 - 40, weiterhin **gekennzeichnet durch**:
vor dem Schritt (b):
Versprühen einer Spülflüssigkeit über dem Gerät aus Düsen innerhalb der Kammer bei einer Temperatur unterhalb etwa 35°C zum Entfernen eines Teils der Ablagerung ohne einer wesentlichen Denaturierung von Proteinen in der Ablagerung.

42. Verfahren nach einem der vorstehenden Ansprüche 32 - 41, weiterhin **gekennzeichnet durch**:
zwischen den Schritten (b) und (c):
Versprühen einer Vorbehandlungslösung über dem Gerät, die wenigstens eines enthält eines Pufferagens und eines Korrosionsinhibitors.

43. Verfahren nach Anspruch 42, weiterhin **dadurch gekennzeichnet, dass**:
die Vorbehandlungslösung ein Pufferagens, einen Korrosionsinhibitor und einen grenzflächenwirksamen Stoff enthält.

44. Verfahren nach einem der vorstehenden Ansprüche 42 und 43, weiterhin **dadurch gekennzeichnet, dass**
nach dem Schritt des Versprühens der Vorbehandlungslösung und vor dem Schritt (c):
Kombinieren der Vorbehandlungslösung mit einem antimikrobiellen dekontaminierenden Mittel zur Bildung der dekontaminierenden Lösung.

45. Verfahren nach einem der vorstehenden Ansprüche 32 - 44, weiterhin **gekennzeichnet durch**:
Anordnen eines zweiten Geräts in einer zweiten Kammer;
Ausführen der Schritte (b), (c) und (d) mit einer gemeinsamen Steuerung unabhängig und asynchron in den beiden Kammern.

46. Verfahren nach einem der vorstehenden Ansprüche 32 - 45, weiterhin **dadurch gekennzeichnet, dass**:
die dekontaminierende Lösung eine Persäure aufweist.

47. Verfahren nach Anspruch 46, weiterhin **gekennzeichnet durch**:
während des Schritts (c):
Überwachen der Konzentration der Persäure in der dekontaminierenden Lösung.

48. Verfahren nach Anspruch 47, weiterhin **dadurch gekennzeichnet, dass**:
wenn die Konzentration der Persäure unterhalb eine vorbestimmte Schwelle fällt, Ausführen wenigstens eines Schrittes aus:
Beenden des Verfahrens,
Signalisieren, dass die Persäure unterhalb der vorbestimmten Schwelle liegt,
Zuführen von zusätzlicher Persäure zu der dekontaminierenden Lösung, und
Ausdehnen des Schrittes (c) für eine ausreichende Zeitdauer zur mikrobiellen Dekontaminierung der Außenflächen.

49. Verfahren nach einem der vorstehenden Ansprüche 32 - 48, weiterhin **dadurch gekennzeichnet, dass**:
der Schritt (a) aufweist:
Einlegen des Geräts in die Kammer durch eine Öffnung (14);
wobei das Verfahren weiterhin aufweist:
Verschließen der Öffnung mit einer Türe (18),
Bewegen eines Klinkenmechanismus (238) aus einer entriegelten Stellung, in der der Klinkenmechanismus ein Öffnen der Türe gestattet, zu einer verriegelten Stellung, in der der Klinkenmechanismus die Türe in einer die Zugangsöffnung abdichtenden Stellung hält, und
Bewegen eines Verriegelungsmechanismus (280) aus einer entriegelten Stellung, in der der Verriegelungsmechanismus ein Entriegeln des Klinkenmechanismus gestattet, zu einer verriegelten Stellung, in der der Verriegelungsmechanismus den Klinkenmechanismus verriegelt zum Verhindern eines Öffnens der Türe.

50. Verfahren nach Anspruch 49, weiterhin **dadurch gekennzeichnet, dass**:
nach dem Schritt der Bewegung des Klinkenmechanismus in die verriegelte Stellung:
ein automatisches Erfassen, dass sich die Türe in der verriegelten Stellung befindet vor der Bewegung des Verriegelungsmechanismus in die Verriegelungsstellung.

51. Verfahren nach Anspruch 50, weiterhin **gekennzeichnet durch**:
Steuern des Verriegelungsmechanismus während der Schritte (b) - (d) zur Sicherstellung, dass sich der Verriegelungsmechanismus nicht in die entriegelte Stellung bewegt, bis der Schritt (d) abgeschlossen ist.

52. Verfahren nach einem der vorstehenden Ansprüche 49 - 51, weiterhin **dadurch gekennzeichnet, dass**:
der Schritt der Bewegung des Klinkenmechanismus in die verriegelte Position aufweist:
Verschwenken wenigstens eines Arms (240, 242, 244, 246), der mit einer Außenseite der Kammer verbunden ist, bis eine drehbar mit dem Arm verbundene Rolle (264) mit einem Eingriffsbauteil (272) an einer Außenfläche der Türe in Eingriff gelangt.

53. Verfahren nach Anspruch 52, weiterhin **dadurch gekennzeichnet, dass**:
der Schritt der Bewegung des Verriegelungsmechanismus in die verriegelte Stellung weiterhin aufweist:
Ergreifen des Arms zum Verhindern eines Verschwenkens des Arms in der verriegelten Stellung.

54. Verfahren nach einem der vorstehenden Ansprüche 49 - 53, weiterhin **gekennzeichnet durch**:
automatisches Erfassen, dass sich der Verriegelungsmechanismus in der verriegelten Stellung befindet vor dem Beginnen des Schrittes (b).

55. Verfahren nach einem der vorstehenden Ansprüche 32 - 54, weiterhin **dadurch gekennzeichnet, dass**:
der Schritt a) aufweist:
Positionieren des Geräts innerhalb der Kammer derart, dass die Sprühstrahlen der Waschlösung und der mikrobiellen dekontaminierenden Lösung aus den Düsen auf Außenflächen des Geräts während der Schritte b) und d) auftreffen, wobei jede der Außenflächen des Geräts mit wenigstens einem Sprühstrahl unter einem Winkel von nicht mehr als 45° aus der Senkrechten zu dem Gerät in Kontakt stehen.

56. Verfahren nach Anspruch 55, weiterhin **dadurch gekennzeichnet, dass**:
der Schritt a) weiterhin aufweist:
Positionieren des Geräts innerhalb der Kammer derart, dass jede der Außenflächen des Geräts nicht weiter als 20 cm von wenigstens einer der Düsen beabstandet ist.

57. Verfahren nach Anspruch 56, weiterhin **dadurch gekennzeichnet, dass**:
der Schritt der Positionierung des Geräts innerhalb der Kammer ein Aufwickeln des Geräts an einem Gestell (21) umfasst.

58. Verfahren nach Anspruch 57, weiterhin **gekennzeichnet durch**:
nach einem mikrobiellen dekontaminierenden Zyklus:
folgt ein Anheben des Gestells aus der Kammer zum Entfernen des Gestells und des Geräts als eine Einheit.

59. Verfahren nach einem der vorstehenden Ansprüche 32 - 58, weiterhin **dadurch gekennzeichnet, dass**:
die Schritte c) und e) weiterhin aufweisen:
Zuführen der Wasch- oder dekontaminierenden Lösung an ein erstes Lumen unter einem ersten Flüssigkeitsdruck, und
Versprühen der Wasch- oder dekontaminierenden Lösung an ein zweites Lumen unter einem zweiten Flüssigkeitsdruck.

60. Verfahren nach einem der vorstehenden Ansprüche 32 - 59, weiterhin **gekennzeichnet durch**:
Durchleiten der Spülflüssigkeit **durch** einen rückspülbaren Abzugsfilter zum Entfernen von Schmutz;
Durchleiten eines Teils der dekontaminierenden Lösung **durch** den rückspülbaren Abzugsfilter zum Dekontaminieren des rückspülbaren Abzugsfilters.

61. Verfahren nach Anspruch 60, weiterhin **gekennzeichnet durch**:
Isolieren des rückspülbaren Abzugsfilters aus der Flüssigkeitsbahn; und
Durchleiten einer verbleibenden Menge der dekontaminierenden Lösung **durch** die Flüssigkeitsbahn.

## Revendications

1. Système automatisé (A) pour le nettoyage et la décontamination microbienne d'un dispositif (B), le système automatisé comprenant :
un coffret (10) qui définit une chambre intérieure (12) pour la réception du dispositif;
des buses de pulvérisation (102, 104, 106, 107, 108, 110) disposées à l'intérieur de la chambre pour la pulvérisation d'un fluide de lavage et d'un fluide de décontamination microbienne sur une surface extérieure (124) du dispositif;
une source (42, 406) d'eau de rinçage;
une source (30, 44, 45) du fluide de lavage;
une source (30, 44, 46, 47) du fluide de décontamination microbienne;
un circuit de distribution de fluides (26) mettant en communication fluidique les sources d'eau de rinçage, de fluide de lavage et de fluide de décontamination microbienne avec les buses;
une pompe (24) connectée au circuit de distribution de fluides pour le pompage du fluide de lavage, du fluide de décontamination microbienne et de l'eau de rinçage vers les buses et qui pompe les solutions pulvérisées vers les buses; et
un système de commande (80) qui régule l'alimentation du fluide de lavage, du fluide de décontamination microbienne et de l'eau de rinçage aux buses de manière à ce que le dispositif soit lavé séquentiellement avec le fluide de lavage, soumis à une décontamination microbienne avec le fluide de décontamination microbienne et rincé avec l'eau de rinçage, le système de commande (80) provoquant séquentiellement une pulvérisation de la solution de décontamination par un premier ensemble (102) des buses de pulvérisation pendant une première période de temps, puis provoquant une pulvérisation de la solution de décontamination par un second ensemble (104) des buses de pulvérisation pendant une seconde période de temps.

2. Système automatisé selon la revendication 1, **caractérisé, en outre par** :
des orifices de raccordement (150, 152, 154) à l'intérieur de la chambre (12), raccordés au circuit de distribution de fluides (26), pour la connexion à un passage intérieur (187) du dispositif (B) de manière à ce que le passage intérieur soit lavé séquentiellement avec le fluide de lavage, soumis à une décontamination microbienne avec le fluide de décontamination microbienne et rincé avec l'eau de rinçage.

3. Système automatisé selon la revendication 2, **caractérisé, en outre par** :
un connecteur (180, 184) pour le raccordement rapide de l'orifice de raccordement (150, 152, 154) au passage intérieur (187) du dispositif (B), le connecteur étant configuré pour laisser fuir une portion du fluide de décontamination microbienne entre le connecteur et une surface adjacente (186) du passage intérieur du dispositif pour assurer le contact entre le fluide de décontamination microbienne et la surface adjacente.

4. Système automatisé selon l'une quelconque des revendications précédentes 1 à 3, **caractérisé, en outre, par** :
un élément d'appui (21) pour soutenir le dispositif à l'intérieur de la chambre; et
un système d'activation (330) qui agite l'élément d'appui pour changer les points de contact entre le dispositif (B) et l'élément d'appui.

5. Système automatisé selon la revendication 4, **caractérisé, en outre, en ce que**:
le système d'activation (330) comprend un piston (344).

6. Système automatisé selon l'une quelconque des revendications précédentes 2 à 5, **caractérisé, en outre, en ce que** :
la source d'eau de rinçage comprend :
un générateur d'eau stérile (406) en communication fluidique avec les buses (102, 104, 106, 107, 108, 110) et avec les orifices de raccordement (150, 152, 154), qui stérilise l'eau non stérile et qui alimente l'eau stérile aux buses et aux orifices de raccordement pour rincer le dispositif, le système de commande (80) régulant le minutage de l'alimentation en eau stérile.

7. Système automatisé selon l'une quelconque des revendications précédentes 1 à 6, **caractérisé, en outre, par** :
une conduite d'air (322) en communication fluidique avec le circuit de distribution de fluides (26) pour alimenter l'air filtré aux buses de pulvérisation (102,104,106,107,108,110) pour sécher le dispositif.

8. Système automatisé selon l'une quelconque des revendications précédentes 1 à 7, **caractérisé, en outre, en ce que** :
la chambre (12) comprend, en outre, une porte (18) qui ferme hermétiquement sélectivement une ouverture d'accès (14) à la chambre, le système de commande (80) empêchant l'ouverture de la porte pendant que les buses (102, 104, 106, 107, 108, 110) pulvérisent le fluide de décontamination sur le dispositif (B).

9. Système automatisé selon l'une quelconque des revendications précédentes 1 à 8, **caractérisé, en outre, par** :
un mécanisme de verrouillage (238) qui se déplace sélectivement d'une position de verrouillage, dans laquelle le mécanisme de verrouillage maintient la porte (18) dans une relation de fermeture hermétique en travers de l'ouverture d'accès (14), dans une position déverrouillée, dans laquelle le mécanisme de verrouillage permet l'ouverture de la porte; et
un mécanisme de blocage (280) qui permet sélectivement le déverrouillage du mécanisme de verrouillage dans une position débloquée et qui bloque le mécanisme de verrouillage dans une position bloquée, empêchant l'ouverture de la porte, le système de commande (80) commandant le mouvement du mécanisme de verrouillage entre les positions débloquée et bloquée.

10. Système automatisé selon la revendication 9, **caractérisé, en outre, en ce que** :
le mécanisme de verrouillage (238) comprend :
un bras (240, 242, 244, 246) raccordé de manière à pivoter, près d'une première extrémité (256) de celui-ci, au coffret (10), et
un galet (264), raccordé au bras de manière mobile autour d'un axe et écarté de la première extrémité, qui enclenche un élément d'enclenchement (272) sur une surface extérieure de la porte dans la position verrouillée.

11. Système automatisé selon la revendication 10, **caractérisé, en outre, en ce que** :
le mécanisme de verrouillage (280) comprend, en outre, :
une tige (282) qui enclenche le bras (242) dans la position bloquée, empêchant le pivotement du bras, et qui permet le pivotement du bras en l'écartant de l'élément d'enclenchement dans la position débloquée, et
un élément d'entraînement (284), l'élément d'entraînement entraînant la tige entre la position débloquée et la position bloquée.

12. Système automatisé selon la revendication 11, **caractérisé, en outre, en ce que** :
l'élément d'entraînement (284) comprend un vérin pneumatique.

13. Système automatisé selon l'une quelconque des revendications précédentes 1 à 12, **caractérisé, en outre, par** :
un orifice de raccordement de détecteur de fuite (202) à l'intérieur de la chambre (12), l'orifice de raccordement de détecteur de fuite étant adapté pour le raccordement à un passage intérieur du dispositif; et
un détecteur de fuite (204) en communication fluidique avec l'orifice de raccordement qui met sous pression le passage intérieur avec un gaz pour la détection de fuites entre une gaine et des lumières du dispositif, le système de commande (80) commandant sélectivement le fonctionnement du détecteur de fuites.

14. Système automatisé selon l'une quelconque des revendications précédentes 1 à 13, **caractérisé, en outre, par** :
au moins un parmi :
un détecteur (398) qui détecte la présence d'un détergent dans le fluide de lavage, et
un détecteur (402) qui détecte la présence d'un décontaminant dans le fluide de décontamination.

15. Système automatisé selon l'une quelconque des revendications précédentes 1 à 14, **caractérisé, en outre, par** :
une paire de coffrets (10, 10') montés l'un à côté de l'autre, chaque coffret définissant une chambre intérieure (12, 12').

16. Système automatisé selon la revendication 15, **caractérisé, en outre, en ce que** :
le système de commande (80) commande la seconde chambre (12') indépendamment de la première chambre (12).

17. Système automatisé selon l'une quelconque des revendications précédentes 1 à 16, **caractérisé, en outre, en ce que** :
les buses (102, 104, 106, 107, 108, 110) et le dispositif (B) sont positionnés à l'intérieur de la chambre (12) de manière ce que la surface extérieure (124) du dispositif soit frappée par une pulvérisation (122) provenant d'au moins une buse selon un angle (θ) ne dépassant pas 45 degrés à partir de la perpendiculaire par rapport à la surface extérieure.

18. Système automatisé selon la revendication 17, **caractérisé, en outre, en ce que** :
les buses (102, 104, 106, 107, 108, 110) et le dispositif (B) sont positionnés à l'intérieur de la chambre (12) de manière ce que toute surface extérieure du dispositif ne se trouve pas à plus de 20 centimètres d'au moins une buse.

19. Système automatisé selon l'une quelconque des revendications précédentes 1 à 18, **caractérisé, en outre, en ce que** :
au moins une première (102, 104, 106, 107, 110) des buses de pulvérisation est positionnée sur une paroi (114, 116, 118) de la chambre (12) et qu'au moins une deuxième (108) des buses de pulvérisation est positionnée sur une porte (18) de la chambre.

20. Système automatisé selon l'une quelconque des revendications précédentes 1 à 19, **caractérisé, en outre, en ce que** :
le système de commande commande l'alimentation aux buses (102, 104, 106, 107, 108, 110) de telle manière que les buses dont les profils de pulvérisation se croisent et interféreraient les uns avec les autres fonctionnent en alternance.

21. Système automatisé selon l'une quelconque des revendications précédentes 1 à 20, **caractérisé, en outre, en ce que** :
les buses comprennent un assemblage de buses (110) disposé à côté d'un centre géométrique de la chambre (10) qui pulvérise dans le sens radial vers l'extérieur.

22. Système automatisé selon la revendication 21, **caractérisé, en outre, en ce que** :
l'assemblage de buses (110) comprend au moins une parmi :
une pluralité de têtes de pulvérisation fixes (121) montées sur un support central et dirigées dans le sens radial vers l'extérieur, et
une buse rotative.

23. Système automatisé selon l'une quelconque des revendications précédentes 1 à 22, **caractérisé, en outre, par** :
un bâti amovible (21) soutenu à l'intérieur de la chambre pour le positionnement du dispositif de manière à ce que la solution de décontamination pulvérisée par les buses (102, 104, 106, 107, 108, 110) vienne en contact avec l'ensemble de la surface extérieure (124) du dispositif.

24. Système automatisé selon la revendication 23, **caractérisé, en outre, en ce que** :
le bâti (21) comprend des éléments (212) qui positionnent le dispositif de manière à ce que les portions flexibles du dispositif soient recourbées pour former un arc ayant un rayon minimal qui n'est pas inférieur à 15 centimètres.

25. Système automatisé selon la revendication 17 ou la revendication 24, **caractérisé, en outre, en ce que** :
le bâti (21) comprend des chevilles qui maintiennent les portions du dispositif difficiles à nettoyer dans des positions reproductibles pour recevoir une pulvérisation optimale des buses.

26. Système automatisé selon l'une quelconque des revendications précédentes 24 et 25, **caractérisé, en outre, par** :
une attache (214, 214') qui relie de manière détachable une portion de pointe du dispositif (B) à l'une du bâti (21) et à une autre portion du dispositif pour maintenir la portion de pointe positionnée fixement.

27. Système automatisé selon la revendication 26, **caractérisé, en outre, en ce que** :
l'attache (214, 214') comprend au moins un doigt supérieur (218, 218') et au moins un doigt inférieur (220, 220'), les doigts étant chacun en contact avec le dispositif au niveau d'une arête (222) pour minimiser le contact entre l'attache et le dispositif.

28. Système automatisé selon la revendication 27, **caractérisé, en outre, en ce que** :
l'attache (214) est adaptée pour l'interconnexion avec le circuit de distribution de fluides (26) et comprend :
des ouvertures (224) définies dans les doigts (218, 218', 220, 220') pour alimenter la solution de décontamination à des portions de l'endoscope mises en contact avec les doigts.

29. Système automatisé selon l'une quelconque des revendications précédentes 1 à 28, **caractérisé, en outre, en ce que** :
le circuit de distribution de fluides (26) comprend :
une première voie de fluide (160, 162) raccordée à un premier orifice de raccordement (150A, 150B),
un premier régulateur (168, 170) régulant la pression de la solution de décontamination s'écoulant à travers la première voie de fluide vers un premier passage intérieur du dispositif,
une seconde voie de fluide (164) raccordée à au moins un second orifice de raccordement (152),
un second régulateur (172) régulant la pression de la solution de décontamination s'écoulant à travers la seconde voie de fluide vers un second passage intérieur du dispositif, de manière à ce que les taux de pression maximaux du premier et du second passage intérieur ne soient pas dépassés.

30. Système automatisé selon l'une quelconque des revendications précédentes 1 à 29, **caractérisé, en outre, par** :
un dispositif de retraitement de valve (158) raccordé au circuit de distribution de fluides (26) pour recevoir une valve d'endoscope (234), le dispositif de retraitement de valve ouvrant la valve tout en faisant couler les solutions sur et à travers la valve.

31. Système automatisé selon l'une quelconque des revendications précédentes 1 à 30, **caractérisé, en outre, par** :
un filtre de drainage lavable à contre-courant (386) raccordé au circuit de distribution de fluides (26) qui enlève les débris grossiers des fluides en circulation.

32. Procédé de nettoyage et de décontamination microbienne d'un dispositif (B) comprenant les étapes séquentielles consistant à :
(a) positionner le dispositif à l'intérieur d'une chambre (12);
(b) pulvériser une solution de lavage sur le dispositif à partir de buses (102, 104, 106, 107, 108, 110) à l'intérieur de la chambre pour enlever les salissures de surfaces extérieures (124) du dispositif;
(c) pulvériser une solution de décontamination microbienne sur le dispositif à partir de buses (102, 104, 106, 107, 108, 110) à l'intérieur de la chambre pour effectuer une décontamination microbienne des surfaces extérieures du dispositif, comprenant :
la pulvérisation alternative avec différents groupes de buses, un premier ensemble (102) des buses de pulvérisation pulvérisant la solution de décontamination pendant une première période de temps, puis un second ensemble (104) des buses de pulvérisation pulvérisant la solution de décontamination pendant une seconde période de temps; et
(d) pulvériser une solution de rinçage sur le dispositif à partir de buses (102, 104, 106, 107, 108, 110) à l'intérieur de la chambre pour rincer les surfaces extérieures du dispositif.

33. Procédé selon la revendication 32, **caractérisé, en outre, en ce que** :
au moins une parmi :
l'étape (b) comprend, en outre, la mise en circulation de la solution de lavage à travers une lumière (187) du dispositif,
l'étape (c) comprend, en outre, la mise en circulation de la solution de décontamination microbienne à travers la lumière du dispositif, et
l'étape (d) comprend, en outre, la mise en circulation du fluide de rinçage à travers la lumière du dispositif.

34. Procédé selon la revendication 33, **caractérisé, en outre, par** :
l'alimentation de la solution de lavage ou de décontamination vers la lumière par l'intermédiaire d'un connecteur de fuite (184) qui permet à une portion des solutions de s'échapper du dispositif autour du connecteur.

35. Procédé selon l'une quelconque des revendications précédentes 33 à 34, **caractérisé, en outre, en ce que** :
l'étape (d) comprend :
la stérilisation de l'eau pour former le fluide de rinçage.

36. Procédé selon l'une quelconque des revendications précédentes 33 à 35, **caractérisé, en outre, par** :
(e) la pulvérisation de l'extérieur de l'endoscope avec de l'alcool et la mise en circulation de l'alcool à travers la lumière, et le drainage de l'alcool.

37. Procédé selon l'une quelconque des revendications précédentes 33 à 36, **caractérisé, en outre, par** :
(e) le soufflage, après l'étape (d), d'air filtré à travers la lumière.

38. Procédé selon l'une quelconque des revendications précédentes 33 à 37, **caractérisé, en outre, par** :
entre les étapes (a) et (b) :
le test d'étanchéité d'au moins un passage intérieur du dispositif, la poursuite de l'étape (b) seulement en réponse à l'approbation du test de fuite.

39. Procédé selon l'une quelconque des revendications précédentes 32 à 37, **caractérisé, en outre, par** :
l'appui du dispositif sur un élément d'appui (21); et
l'agitation de l'élément d'appui pour changer les points de contact entre le dispositif (B) et l'élément d'appui.

40. Procédé selon la revendication 39, **caractérisé par** :
l'étape d'agitation de l'élément d'appui comprenant :
le frappement de l'élément d'appui à des intervalles de manière à ce que l'élément d'appui pivote autour d'un point de pivotement (210).

41. Procédé selon l'une quelconque des revendications précédentes 32 à 40, **caractérisé, en outre, par** :
avant l'étape (b) :
la pulvérisation d'un liquide de rinçage sur le dispositif à partir des buses à l'intérieur de la chambre à une température inférieure à environ 35°C pour enlever une portion des salissures sans dénaturer sensiblement les protéines dans les salissures.

42. Procédé selon l'une quelconque des revendications précédentes 32 à 41, **caractérisé, en outre, par** :
entre les étapes (b) et (c) :
la pulvérisation d'une solution de pré-traitement sur le dispositif qui comprend au moins un parmi un tampon et un inhibiteur de corrosion.

43. Procédé selon la revendication 42, **caractérisé, en outre, en ce que** :
la solution de pré-traitement comprend un tampon, un inhibiteur de corrosion et un tensioactif.

44. Procédé selon l'une quelconque des revendications précédentes 42 et 43, **caractérisé, en outre, en ce que** :
après l'étape de pulvérisation de la solution de pré-traitement et avant l'étape (c) :
la solution de pré-traitement est combinée avec un décontaminant microbien pour former la solution de décontamination.

45. Procédé selon l'une quelconque des revendications précédentes 32 à 44, **caractérisé, en outre, par** :
le montage d'un second dispositif dans une seconde chambre;
avec un régulateur commun, permettant de réaliser les étapes (b), (c) et (d) de manière indépendante et asynchrone dans les deux chambres.

46. Procédé selon l'une quelconque des revendications précédentes 32 à 45, **caractérisé, en outre, en ce que** :
la solution de décontamination contient de l'acide peracétique.

47. Procédé selon la revendication 46, **caractérisé, en outre, par** :
pendant l'étape (c) :
la surveillance de la concentration en acide peracétique dans la solution de décontamination.

48. Procédé selon la revendication 47, **caractérisé, en outre, en ce que** :
si la concentration en acide peracétique est inférieure à un niveau prédéterminé, on réalise au moins une des opérations suivantes :
complétion du procédé,
indication que l'acide peracétique est inférieur au niveau prédéterminé,
ajout d'acide peracétique additionnel dans la solution de décontamination, et
poursuite de l'étape (c) pendant une période de temps suffisante pour effectuer une décontamination microbienne des surfaces extérieures.

49. Procédé selon l'une quelconque des revendications précédentes 32 à 48, **caractérisé, en outre, en ce que** :
l'étape (a) comprend :
le chargement du dispositif dans la chambre à travers une ouverture (14);
et **en ce que** le procédé comprend :
la fermeture hermétique de l'ouverture avec une porte (18),
le déplacement d'un mécanisme de verrouillage (238) d'une position de déverrouillage, dans laquelle le mécanisme de verrouillage permet l'ouverture de la porte dans une position de verrouillage, dans laquelle le mécanisme de verrouillage maintient la porte dans une relation de fermeture hermétique en travers de l'ouverture d'accès, et
le déplacement d'un mécanisme de blocage (280) d'une position débloquée, dans laquelle le mécanisme de blocage permet le déverrouillage du mécanisme de verrouillage, dans une position bloquée, dans laquelle le mécanisme de blocage bloque le mécanisme de verrouillage, empêchant l'ouverture de la porte.

50. Procédé selon la revendication 49, **caractérisé, en outre, en ce que** :
après l'étape de déplacement du mécanisme de verrouillage dans la position de verrouillage :
on détecte automatiquement que la porte est dans la position de verrouillage avant de déplacer le mécanisme de verrouillage dans la position bloquée.

51. Procédé selon la revendication 50, **caractérisé, en outre, en ce que** :
le mécanisme de blocage est commandé pendant les étapes (b) à (d) pour s'assurer que le mécanisme de blocage ne se déplace pas dans la position de déblocage jusqu'à ce que l'étape (d) soit terminée.

52. Procédé selon l'une quelconque des revendications précédentes 49 à 51, **caractérisé, en outre, par** :
l'étape de déplacement du mécanisme de verrouillage dans la position de verrouillage, comprenant :
le pivotement d'au moins un bras (240, 242, 244, 246) raccordé à une face extérieure de la chambre jusqu'à ce qu'un galet (264), raccordé au bras de manière mobile autour d'un axe, enclenche un élément d'enclenchement (272) sur une surface extérieure de la porte.

53. Procédé selon la revendication 52, **caractérisé, en outre, en ce que** :
l'étape de déplacement du mécanisme de blocage dans la position bloquée comprend, en outre, :
l'enclenchement de l'arbre pour empêcher le pivotement du bras dans la position bloquée.

54. Procédé selon l'une quelconque des revendications précédentes 49 à 53, **caractérisé, en outre, en ce que** :
l'on détecte automatiquement que le mécanisme de blocage est dans la position bloquée avant de démarrer l'étape (b).

55. Procédé selon l'une quelconque des revendications précédentes 32 à 54, **caractérisé, en outre, en ce que** :
l'étape a) comprend :
le positionnement du dispositif à l'intérieur de la chambre de manière à ce que les jets de pulvérisation de la solution de la lavage et de la solution de décontamination microbienne provenant des buses frappent les surfaces extérieures du dispositif pendant les étapes b) et d), chacune de surfaces extérieures du dispositif étant mise au contact d'au moins un jet de pulvérisation selon un angle ne dépassant pas 45 degrés à partir de la perpendiculaire par rapport au dispositif.

56. Procédé selon la revendication 55, **caractérisé, en outre, en ce que** :
l'étape a) comprend, en outre, :
le positionnement du dispositif à l'intérieur de la chambre de manière à ce que chacune des surfaces extérieures du dispositif ne se trouve pas à plus de 20 centimètres d'au moins l'une des buses.

57. Procédé selon la revendication 56, **caractérisé, en outre, en ce que** :
l'étape de positionnement du dispositif à l'intérieur de la chambre comprend l'enroulement du dispositif sur un bâti (21).

58. Procédé selon la revendication 57, **caractérisé, en outre, par** : après le cycle de décontamination microbienne :
le levage du bâti hors de la chambre pour enlever le bâti et le dispositif en un ensemble.

59. Procédé selon l'une quelconque des revendications précédentes 32 à 58, **caractérisé, en outre, en ce que** :
les étapes c) et e) comprennent, en outre, :
l'alimentation de la solution de lavage ou de décontamination à une première lumière sous une première pression de fluide, et
l'alimentation de la solution de lavage ou de décontamination à une seconde lumière sous une seconde pression de fluide.

60. Procédé selon l'une quelconque des revendications précédentes 32 à 59, **caractérisé, en outre, en ce que** :
l'on fait passer le fluide de rinçage à travers un filtre de drainage lavable à contre-courant pour enlever les débris grossiers;
l'on fait passer une portion de la solution de décontamination à travers le filtre de drainage lavable à contre-courant pour décontaminer le filtre de drainage lavable à contre-courant.

61. Procédé selon la revendication 60, **caractérisé, en outre, en ce que** :
l'on isole le filtre de drainage lavable à contre-courant de la voie de fluide; et
l'on fait passer une portion restante de la solution de décontamination à travers la voie de fluide.
